# EUROPEAN PATENT APPLICATION

(11) **EP 4 687 149 A1**
(43) Date of publication of application: **04.02.2026**
(21) Application number: 25188533.1
(22) Date of filing: 09.07.2025
(51) Int. Cl.: G16H 20/40, A61M 16/00, G06T 7/00, G06T 7/136, G16H 30/40, G16H 50/70, G16H 70/60

(54) **METHODS AND SYSTEMS FOR VENTILATION SYSTEM MONITORING**

(30) Priority: 31.07.2024 US 202418791195
(71) Applicant: GE Precision Healthcare LLC, Waukesha, WI 53188 (US)
(72) Inventor: Nandakumar, J R, 560066 Bengaluru (IN); Kshireswar, BHOI, 560066 Bengaluru (IN); HÄGGBLOM, Tom Jakob, 00510 Helsink (FI)
(74) Representative: Kilburn & Strode LLP

(57) **Abstract**

Methods and systems are provided for a ventilation system (100). In one example, a method includes obtaining (504, 602) one or more patient ventilation parameter images of a patient with the ventilation system while the patient is undergoing mechanical ventilation; obtaining one or more reference ventilation parameter images; processing, with at least one comparison model, each patient ventilation parameter image and each reference ventilation parameter image to characterize at least one feature in each patient ventilation parameter image and each reference ventilation parameter image, including converting each patient ventilation parameter image and each reference ventilation parameter image to a binary mask; identifying (508, 606), based on of the at least one feature, a deviation between a patient ventilation parameter image and a corresponding reference ventilation parameter image; and in response to the identifying, outputting (510, 608) a notification that indicates the deviation.

## Description

### FIELD

Embodiments of the subject matter disclosed herein relate to assisted ventilation of a subject.

### BACKGROUND

During an event where ventilation support is demanded for a subject, such as a patient, undergoing a surgery or a procedure which requires anesthetization, a ventilation system may be used to provide requisite pulmonary gas exchanges. The ventilation system may have a relatively complex configuration for delivering oxygen to and removing carbon dioxide from the subject's lungs and may rely on microprocessor-based control of sensors, valves, flow rate controllers, and various other components. The subject may thereby be mechanically ventilated and flow of gases to and from the subject may be monitored and controlled by the ventilation system and one or more clinicians, such as an anesthesiologist.

### BRIEF DESCRIPTION

In one embodiment, a method for a ventilation system includes obtaining one or more patient ventilation parameter images of a patient with the ventilation system while the patient is undergoing mechanical ventilation with the ventilation system; obtaining one or more reference ventilation parameter images; processing, with at least one comparison model, each patient ventilation parameter image and each reference ventilation parameter image to characterize at least one feature in each patient ventilation parameter image and each reference ventilation parameter image, the processing including converting each patient ventilation parameter image and each reference ventilation parameter image to a binary mask; identifying, based on the at least one feature in each patient ventilation parameter image and each reference ventilation parameter image, a deviation between a patient ventilation parameter image and a corresponding reference ventilation parameter image; and in response to the identifying, outputting a notification that indicates the deviation.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows an example of a medical system for providing respiratory support to a subject.
FIG. 2 shows a block diagram of the medical system of FIG. 1.
FIG. 3 shows a block diagram of a ventilation system including the medical system of FIGS. 1-2.
FIG. 4 is a flow chart illustrating a high-level method for monitoring a ventilation system.
FIG. 5 is a flow chart illustrating a method for monitoring the ventilation system using initial patient waveform and spirometry images relative to annotated images via a plurality of comparison models.
FIG. 6 is a flow chart illustrating a method for monitoring the ventilation system using subsequent patient waveform and spirometry images relative to baseline images via the plurality of comparison models.
FIG. 7 is a flow chart illustrating a method for a weaning protocol for confirming the patient is ready to be weaned from the ventilation system.
FIG. 8 is an example graphical user interface that may be displayed on a device of the ventilation system during the execution of the methods of FIGS. 4-7.
FIGS. 9-16 show example spirometry images that may be analyzed via the comparison models disclosed herein.
FIGS. 17-21 show example waveform images that may be analyzed via the comparison models disclosed herein.

### DETAILED DESCRIPTION

The following description relates to various embodiments of a ventilation system, which may include an anesthesia machine and ventilator. During certain procedures, or based on certain patient conditions, a patient may not be able to breathe spontaneously with sufficient gas exchange and thus mechanical ventilation via the ventilation system may be provided. During mechanical ventilation, various patient and machine parameters are measured and displayed for one or more clinicians overseeing the patient, such as respiratory rate (RR), positive end expiratory pressure (PEEP), fractional inspired oxygen (F_{I}O₂), fractional expired oxygen (F_{E}O₂), breathing gas flow (F), tidal volumes (V_{T}), temperatures (T), airway pressures (P_{aw}), and the like. Some parameters may be displayed as time-series data in the form of waveforms, such as pressure, flow, and volume waveforms. Further, some parameters may be combined into spirometry diagrams or images, such as pressure/flow and flow/volume spirometry images, that are also displayed. The clinicians overseeing the patient may monitor the displayed parameters to determine if the patient is stable and the ventilation system is operating as expected. If a clinician notices any deviations from expected parameters, the clinician may initiate various actions, such as adjusting settings of the ventilation system.

However, the above described approach assumes that all clinicians can properly interpret all of the parameters that are displayed. In the case of spirometry images in particular, many clinicians are unable to properly interpret each type of spirometry image and may be unable to identify changes in the spirometry images that are suggestive of potential issues such as a leaking laryngeal mask, a kinked endotracheal tube, or other issues. Further, even for clinicians that can properly interpret all of the displayed parameters, it may be practically impossible for a given clinician to constantly monitor all of the various parameters and recognize deviations suggestive of the above-mentioned issues, as changes in the parameters may occur slowly (and hence be difficult to recognize) or may present differently for different types of patients and different ventilation system settings. For example, a clinician may not know or remember the baseline/normal for each parameter for a given patient at a given set of ventilation system settings and thus may be unable to recognize when any parameters have changed, let alone when the parameters have changed to the point of suggesting an action needs to be taken. Because most clinicians, particularly supervising anesthesiologists or other highly-trained physicians, may be overseeing multiple patients at one time, the above-described issues may be compounded and a clinician may not recognize issues as soon as would be desired.

Thus, the embodiments disclosed herein address these issues by performing automated comparisons between predefined baseline or ground truth ventilation parameter images (e.g., waveform and spirometry images) and current patient ventilation parameter images using one or more comparison models that are configured to identify and quantify various features in ventilation parameter images and output any detected changes in the features. At the initial stages of mechanical ventilation (e.g., when a patient is initially intubated), ground truth waveform and spirometry images may be obtained from a database of annotated waveform and spirometry images that reflect real or simulated waveform and spirometry images generated for patients of various demographics (age, gender, body type, and so forth), as well as at various ventilation system settings. The annotated waveform and spirometry images may be annotated to reflect whether the real or simulated patient was exhibiting satisfactory or unsatisfactory lung performance and whether the ventilation system was exhibiting normal or degraded performance. The initial waveform and spirometry images of the patient may be compared with demographic- and ventilation system parameter-matched annotated waveform and spirometry images using the one or more comparison models to identify if the initial waveform and spirometry images of the patient deviate in any significant way from the annotated waveform and spirometry images, which may indicate an issue with the initial ventilation system settings, for example.

After the initial waveform and spirometry images of the patient are obtained, assuming the initial waveform and spirometry images do not deviate from the matched annotated waveform and spirometry images, the initial waveform and spirometry images of the patient may be saved as baseline images that are used for comparison to subsequent waveform and spirometry images of the patient. If any deviations are detected, one or more clinicians may be notified via displayed alerts, for example, which may prompt the one or more clinicians to adjust the settings of the ventilation system. The prior baseline waveform and spirometry images may be replaced with the current waveform and spirometry images as a new means for comparison (assuming the changed ventilation system settings reflect the ideal for the patient). Each time baseline waveform and spirometry images are replaced, the prior baseline waveform and spirometry images may be moved to on-premise or remote storage to allow further refinement of the one or more comparison models.

The one or more comparison models may also be configured to detect whether the patient is spontaneously breathing or has stopped breathing. If the one or more comparison models detect that the patient is spontaneously breathing, a weaning protocol may be initiated. The weaning protocol may include analysis, via one or more models of the one or more comparison models, of patient video/image frames to determine if the patient is able to follow commands, as well as a rules-based check of patient monitoring parameters such as blood oxygen saturation and heart rate. If the patient is able to follow commands and the parameters pass the rules-based check, the one or more clinicians may be notified that weaning of the patient is recommended.

The above-described approach may therefore allow monitoring of a plurality of parameters of a patient that is undergoing mechanical ventilation, wherein the plurality of parameters may be monitored simultaneously and at a higher rate than feasible if relying on only a clinician to monitor the patient. Subtle or rare deviations in waveforms and/or spirometry images may be detected, using the patient's own waveform and spirometry images as a baseline. In doing so, patient status may be monitored automatically and any changes in the patient status may be identified and the clinicians monitoring the patient alerted to the changes in patient status. Any time that changes are made to the settings of the ventilation system, the baseline images may be automatically updated, so that changes in patient status are detected relative to the most appropriate images, which may reduce instances of unnecessary alerts and improve the efficiency of the monitoring.

Before further discussion of the approach for systems and methods for automatic monitoring of a ventilation system and a patient undergoing mechanical ventilation via the ventilation system, a general description of a medical system configured to provide ventilation support is provided. The figures illustrate diagrams of the functional blocks of various embodiments. The functional blocks are not necessarily indicative of the division between hardware circuitry. Thus, for example, one or more of the functional blocks (e.g., processors or memories) may be implemented in a single piece of hardware (e.g., a general purpose signal processor or a block or random access memory, hard disk, or the like) or multiple pieces of hardware. Similarly, the programs may be stand-alone programs, may be incorporated as subroutines in an operating system, may be functions in an installed software package, and the like. It should be understood that the various embodiments are not limited to the arrangements and instrumentality shown in the drawings.

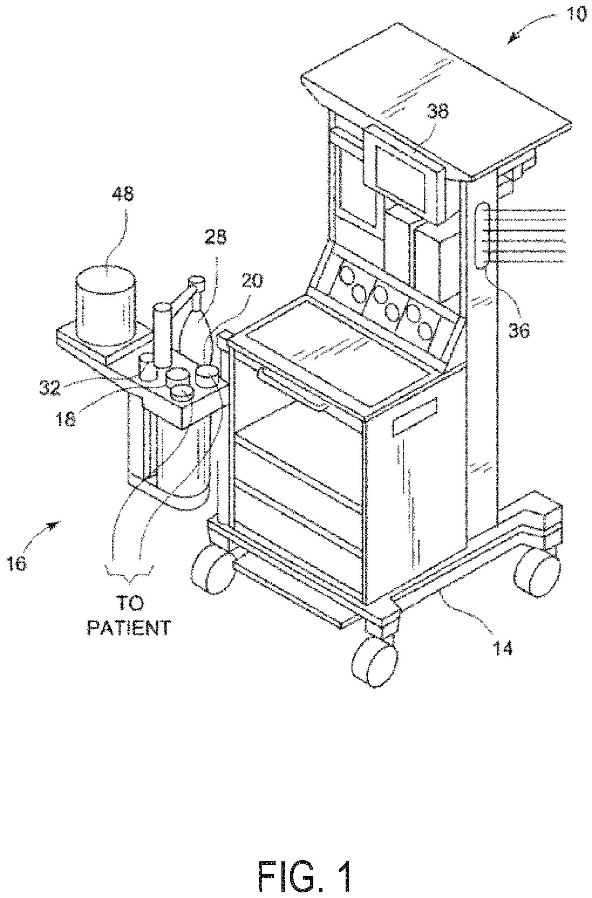

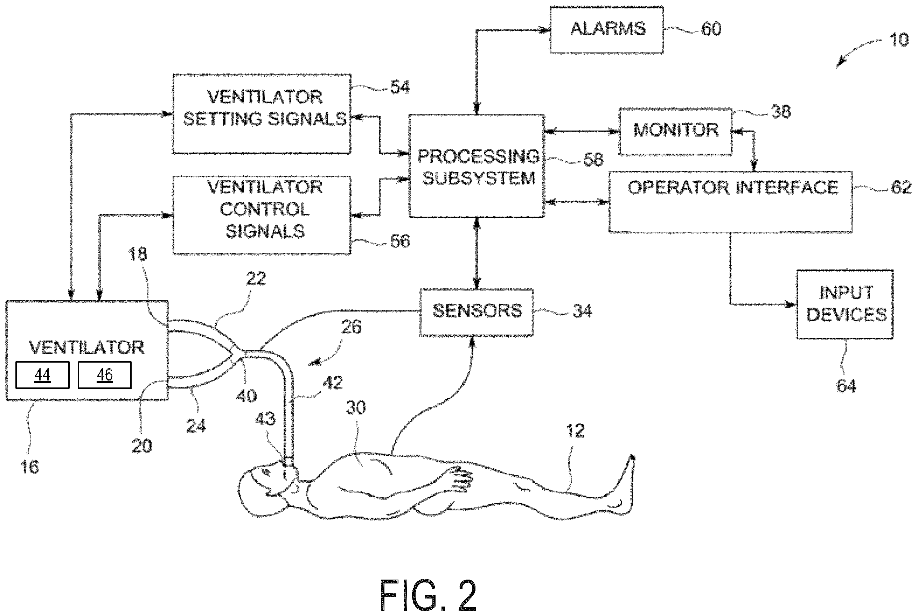

For example, a medical system 10 is shown in FIGS. 1-2, which may include an anesthesia machine 14 with a ventilator 16. As such, the medical system 10 may be referred to as a ventilator and/or an anesthesia machine. The ventilator 16 may have suitable connectors, such as a first connector 18 and a second connector 20, for connecting to an inspiratory branch 22 and expiratory branch 24 of a breathing circuit 26 leading to a patient 12, where the inspiratory branch 22, the expiratory branch 24, the breathing circuit 26, and the patient 12 are depicted in FIG. 2. The ventilator 16 and breathing circuit 26 may cooperate to provide breathing gases to the patient 12 via the inspiratory branch 22 and to receive gases expired by the patient 12 via the expiratory branch 24.

The ventilator 16 may also be provided with a manual resuscitator 28, as shown in FIG. 1, for manually ventilating the patient 12. In one example, the manual resuscitator 28 may be a bag valve mask (BVM), which may also be referred to as a bag. For example, the manual resuscitator 28 may be filled with breathing gases, such as oxygen, anesthesia gases, etc., and be manually squeezed by a clinician (not shown) to provide the breathing gases to the patient 12. Using this manual resuscitator 28, or "bagging the patient," enables clinicians to manually and/or immediately control delivery of the breathing gases to the patient 12. The clinician may also sense conditions in the respiration and/or lungs 30 (as shown in FIG. 2) of the patient 12 according to a sensory feedback during manipulation of the manual resuscitator 28, and adjust manual ventilation accordingly. The ventilator 16 may also provide a bag-to-ventilation (BTV) switch 32 for switching and/or alternating between manual and automated (e.g., mechanical) ventilation when the manual resuscitator 28 is provided.

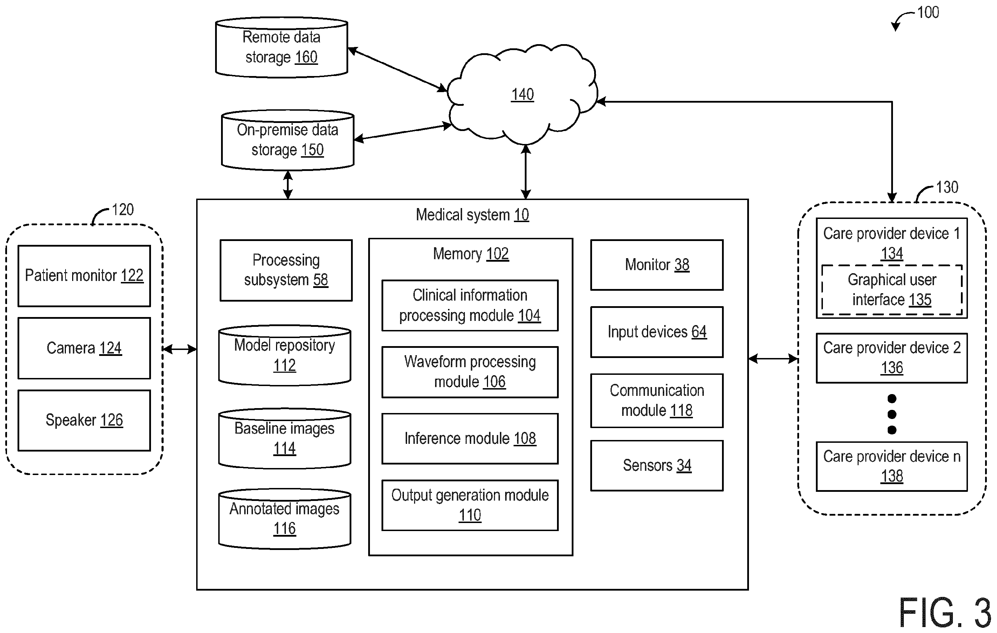

The medical system 10, and particularly a processing terminal 36 and/or a processing subsystem 58 of the medical system 10 (which may be implemented on the anesthesia machine 14), may receive inputs from sensors 34, as shown in FIG. 2, associated with the ventilator 16 and anesthesia machine 14 for subsequent processing thereof. The processed inputs may be displayed on a monitor 38 (e.g., of the anesthesia machine 14). Representative data received from the sensors 34 may include, for example, inspiratory time (T_{I}), expiratory time (T_{E}), natural exhalation time (T_{EXH}), respiratory rates (RR), inspiratory time to expiratory time (I:E) ratios, positive end expiratory pressure (PEEP), fractional inspired oxygen (F_{I}O₂), fractional expired oxygen (F_{E}O₂), breathing gas flow (F), tidal volumes (V_{T}), temperatures (T), airway pressures (P_{aw}), arterial blood oxygen saturation levels (SₐO₂), blood pressure information (BP), pulse rates (PR), pulse oximetry levels (SₚO₂), exhaled CO₂ levels (F_{ET}CO₂), concentration of inspired inhalation anesthetic agent (C₁ agent), concentration of expired inhalation anesthetic agent (C_{E} agent), arterial blood oxygen partial pressure (PₐO₂), arterial carbon dioxide partial pressure (PₐCO₂), and the like. Thus, the sensors 34 may be included in the breathing circuit 26, the ventilator 16, the anesthesia machine 14, and/or on the patient 12. For example, the patient 12 may be connected to a patient monitor that includes sensors to detect BP, PR, and SₚO₂ as shown in FIG. 3 and explained in more detail below.

With particular reference now to FIGS. 2, the ventilator 16 provides breathing gases to the patient 12 via the breathing circuit 26. Accordingly, the breathing circuit 26 includes the inspiratory branch 22 and expiratory branch 24. In some examples, one end of each of the inspiratory branch 22 and expiratory branch 24 is connected to the ventilator 16, while the other ends thereof are connected to a Y-connector 40, which can then connect to the patient 12 through a patient branch 42. An interface 43 also may be provided to secure the patient's 12 airways to the breathing circuit 26 and/or prevent gas leakage out thereof.

The ventilator 16 may also include electronic control circuitry 44 and/or pneumatic circuitry 46. In particular, various pneumatic elements of the pneumatic circuitry 46 may provide breathing gases to the lungs 30 of the patient 12 through the inspiratory branch 22 of the breathing circuit 26 during inhalation. Upon exhalation, the breathing gases may be discharged from the lungs 30 of the patient 12 and into the expiratory branch 24 of the breathing circuit 26. This process can be iteratively enabled by the electronic control circuitry 44 and/or pneumatic circuitry 46 in the ventilator 16, which can establish various control parameters, such as the number of breaths per minute to administer to the patient 12, tidal volumes (V_{T}), maximum pressures, etc., that can characterize the mechanical ventilation that the ventilator 16 supplies to the patient 12. As such, the ventilator 16 may be microprocessor-based and operable in conjunction with a suitable memory to control pulmonary gas exchanges in the breathing circuit 26 connected to, and between, the patient 12 and the ventilator 16.

The various pneumatic elements of the pneumatic circuitry 46 may also include a source of pressurized gas (not shown), which can operate through a gas concentration subsystem (not shown) to provide the breathing gases to the lungs 30 of the patient 12. The pneumatic circuitry 46 may provide the breathing gases directly to the lungs 30 of the patient 12, as may be used in a chronic and/or critical care application, or the pneumatic circuitry 46 may provide a driving gas to compress a bellows 48 (as shown in FIG. 1) containing the breathing gases. In turn, the bellows 48 may supply the breathing gases to the lungs 30 of the patient 12, such as in an anesthesia application. In either case, the breathing gases may iteratively pass from the inspiratory branch 22, to the Y-connector 40 and to the patient 12, and then back to the ventilator 16 via the Y-connector 40 and expiratory branch 24.

In the embodiment illustrated in FIGS. 1-2, one or more of the sensors 34, when placed in the breathing circuit 26, may also provide feedback signals back to the electronic control circuitry 44 of the ventilator 16 via a feedback loop. More specifically, a signal in the feedback loop may be proportional, for example, to gas flows and/or airway pressures in the patient branch 42 leading to the lungs 30 of the patient 12. Inhaled and exhaled gas concentrations (e.g., oxygen (O₂), carbon dioxide (CO₂), nitrous oxide (N₂O), and inhalation anesthetic agents), flow rates (including, for example, spirometry), and gas pressurization levels, etc., may be captured by the sensors 34, as well as durations of time periods between when the ventilator 16 permits the patient 12 to inhale and exhale, and when the patient's natural inspiratory and expiratory flows cease.

Thus, the electronic control circuitry 44 of the ventilator 16 may also control displaying numerical and/or graphical information from the breathing circuit 26 on the monitor 38 of the medical system 10 (as shown in FIGS. 1-2), as well as other patient 12 and/or system 10 parameters from other sensors 34 and/or the processing terminal 36 (as shown in FIG. 1). In other embodiments, various components can also be integrated and/or separated, as needed and/or desired.

The processing subsystem 58 may be located at the processing terminal 36 of FIG. 1 and may include various electronic components, e.g., hardware, for receiving and transmitting signals, and processing thereof. For example, the processing subsystem 58 may include a controller (e.g., a processor) configured to receive signals from the sensors 34, which may include pressure sensors, flow sensors, sensors monitoring statuses of valves and switches, etc., and send control signals to actuators of the medical system, such as pressure regulators, the valves and switches, etc., in response to the sensor signals. The controller may be a microcomputer, including a microprocessor unit, input/output ports, an electronic storage medium (including non-transitory memory) for storing executable programs and parameter setting values. The controller may be programmed with computer readable data representing instructions executable to perform the methods described herein as well as other variants that are anticipated but not specifically listed.

The processing subsystem 58 may also coordinate and/or control, for example, elements depicted in FIG. 2, including ventilator setting signals 54 and ventilator control signals 56. The ventilator setting signals 54 and ventilator control signals 56 may be used to control operation of the ventilator 16 via the electronic control circuitry 44 and the pneumatic circuitry 46. The processing subsystem 58 may further be configured to output signals for display on the monitor 38 and/or the like, control/display alarms 60, and/or control/generate/display an operator interface 62, which may include a graphical user interface (GUI) displayed at the monitor 38, and one or more input devices 64, etc., all as demanded and/or desired and interconnected suitably.

The components of FIGS. 1-2 are depicted for illustration, wherein various other components may also be integrated and/or separated therein, based on demand and/or as desired. Other components, for example, one or more power supplies for the medical system 10 and/or anesthesia machine 14 and/or ventilator 16, etc. (not shown) may be provided.

FIG. 3 shows a block diagram of a ventilation monitoring system 100 including medical system 10. As explained above, medical system 10 includes a monitor 38 (e.g., a display device), input devices 64, sensors 34, and processing subsystem 58. Processing subsystem 58 may be configured to execute instructions stored in memory 102 in order to carry out the various functions described above, as well as the methods disclosed herein. Memory 102 may store a clinical information processing module 104, a waveform processing module 106, an inference module 108, and an output generation module 110. Medical system 10 may further include data storage, including a model repository 112, baseline images 114, and annotated images 116.

Within ventilation monitoring system 100, the medical system 10 may be operatively/communicatively coupled to various devices, whether directly or via a network, such as network 140. As shown in FIG. 3, a plurality of care provider devices 130 may be included as part of network 140 and/or directly coupled to medical system 10, from a first care provider device 134, a second care provider device 136, and on up to an nth care provider device 138. Each care provider device may include a processor, memory, communication module, user input device, display (e.g., screen or monitor), and/or other subsystems and may be in the form of a desktop computing device, a laptop computing device, a tablet, a smart phone, or other device. Each care provider device may be adapted to send and receive encrypted data and display medical information, including medical images in a suitable format such as digital imaging and communications in medicine (DICOM) or other standards. The care provider devices may be located locally at the same medical facility as the medical system 10 and substantially fixed in place (such as in a nurses' station or in the room of a patient) and/or located locally or remotely from the medical facility and configured to move with the care provider (such as a care provider's mobile device).

Medical system 10 may be communicatively coupled to one or more external patient devices 120. The external patient devices 120 may include a patient monitor 122, a camera 124, and/or a speaker 126. The patient monitor 122 may include sensors configured to measure patient status, not included as part of medical system 10, such as sensors configured to measure one or more of oxygen saturation measurement (SpO2), heart rate (HR), and pulse rate (PR). While the external patient devices 120 are shown as being directly coupled to the medical system 10, it is to be appreciated that one or more of the external patient devices may be communicatively coupled to the medical system 10 via network 140. Medical system 10 may further be communicatively coupled to data storage located separately from the medical system 10, such as on-premise data storage 150 and/or remote data storage 160.

The devices disclosed herein, such as the care provider devices and/or aspects of the medical system 10, may each include a communication module, memory, and processor(s) to store and execute the methods disclosed herein as well as send and receive communications, graphical user interfaces, medical data, and other information. For example, medical system 10 may include a communication module 118.

Each communication module facilitates transmission of electronic data within and/or among one or more systems. Communication via the communication module can be implemented using one or more protocols. In some examples, communication via the communication module occurs according to one or more standards (e.g., Digital Imaging and Communications in Medicine (DICOM), Health Level Seven (HL7), ANSI X12N, etc.). The communication module can be a wired interface (e.g., a data bus, a Universal Serial Bus (USB) connection, etc.) and/or a wireless interface (e.g., radio frequency, infrared, near field communication (NFC), etc.). For example, a communication module may communicate via wired local area network (LAN), wireless LAN, wide area network (WAN), etc. using any past, present, or future communication protocol (e.g., BLUETOOTH^{®}, USB 2.0, USB 3.0, etc.).

Each memory (including memory 102, memory of each care provider device, on-premise data storage 150, and remote data storage 160) may include one or more data storage structures, such as optical memory devices, magnetic memory devices, or solid-state memory devices, for storing programs and routines executed by the processor(s) to carry out various functionalities disclosed herein, as well as for storing annotated waveform images, annotated spirometry images, baseline waveform images, and baseline spirometry images, as discussed in more detail below. Memory may include any desired type of volatile and/or non-volatile memory such as, for example, static random access memory (SRAM), dynamic random access memory (DRAM), flash memory, read-only memory (ROM), etc. The processor(s) may be any suitable processor, processing unit, or microprocessor, for example. The processor(s) may be a multiprocessor system, and, thus, may include one or more additional processors that are identical or similar to each other and that are communicatively coupled via an interconnection bus.

As used herein, the terms "sensor," "system," "unit," or "module" may include a hardware and/or software system that operates to perform one or more functions. For example, a sensor, module, unit, or system may include a computer processor, controller, or other logic-based device that performs operations based on instructions stored on a tangible and non-transitory computer readable storage medium, such as a computer memory. Alternatively, a sensor, module, unit, or system may include a hard-wired device that performs operations based on hard-wired logic of the device. Various modules or units shown in the attached figures may represent the hardware that operates based on software or hardwired instructions, the software that directs hardware to perform the operations, or a combination thereof.

"Systems," "units," "sensors," or "modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally or alternatively, one or more of these devices may be hard-wired with logic circuits to perform these operations.

One or more of the devices described herein may be implemented over a cloud or other computer network. For example, model repository 112 and inference module 108 are shown as residing on medical system 10, but it is to be understood that aspects of medical system 10 (e.g., model repository 112 and/or inference module 108) may be implemented on a remote device (e.g., a server) or distributed across multiple devices, such as across multiple servers.

As will be explained in more detail below, ventilation monitoring system 100 may be configured to monitor patient ventilation parameter images, such as waveform images and spirometry images, generated for a patient undergoing mechanical ventilation via medical system 10. The monitoring may include comparing the patient ventilation parameter images to annotated, demographic- and system settings-matched ventilation parameter images to identify any deviations in the patient ventilation parameter images to expected (e.g., ground truth) ventilation parameter images. For example, ventilation waveform and spirometry images may be collected for a wide range of patient population, from neonatal to adults, at different ventilation settings (such as different airway resistance, peak inspiratory pressure (Ppeak), Plateau pressure (Pplat), Positive End Expiratory Pressure (PEEP), Tidal volume (TV), Respiratory Rate (RR), Inspiratory to expiratory time ratio (I:E), etc.). These waveforms and spirometry images may be annotated by expert anesthesiologist/clinicians and stored in an image repository, such as on the cloud or on a server. The annotated waveform and spirometry images may be categorized based on different patient lung conditions and patient breathing circuit conditions, such as good lung and with a good patient breathing circuit (e.g., without any issues such as leak, endotracheal tube (ET tube) blockage, expiratory limb blockage etc.), good lung with a defect in the patient breathing circuit, good lung at weaning phase with spontaneous breathing with adequate tidal volume, and patient not breathing spontaneously. These annotated waveform and spirometry images may be used during the initial phase of ventilation support to detect any deviations from expected patient status by comparing the patient waveform and spirometry images with the annotated waveform and spirometry images via the suite of comparison models.

For example, at the start of mechanical ventilation for the patient, an anesthesiologist may set the ventilation settings for the medical system 10 based on the patient condition (e.g., based on patient information, such as age, gender, height, and weight, and based on the patient diagnosis, reason for the mechanical ventilation, etc.). The medical system 10 may include the clinical information processing module 104, which may be configured to receive patient information from various sources (e.g., an electronic medical record system, a hospital information system, and/or user input via the input devices 64) and process the information to determine the patient condition. Waveform images and spirometry loop images of the patient for the given set of ventilation settings may be generated at a specified time interval (e.g., new waveform and spirometry images may be generated every two minutes) by the waveform processing module 106. The inference module 108 may invoke the one or more comparison models, which may be stored in the model repository 112, to compare the patient waveform and spirometry images with appropriate images from the annotated waveform and spirometry images. In some examples, the annotated waveform and spirometry images may be stored locally on the medical system 10, as part of annotated images 116, or the annotated waveform and spirometry images may be stored remotely and accessed at the time of comparison.

If there are significant deviations observed between the patient waveform and spirometry images and the annotated waveform and spirometry images, then based on the deviations, a notification may be output to the anesthesiologist describing the issue and, when known, possible causes for the issue. For example, the output generation module 110 may generate a pop-up message that may be displayed on the monitor 38 and/or one or more of the care provider devices (such as via a graphical user interface 135). Additionally, the patient waveform and spirometry images may be sent to a designated storage location (e.g., on-premise data storage 150 and/or remote data storage 160) to be used as annotated waveform and spirometry images, for example. When a significant deviation is observed between the patient waveform and spirometry images and the annotated waveform and spirometry images, the anesthesiologist may take action to correct these observations, such as by adjusting settings of the medical system 10, replacing a leaking component, unkinking an ET, etc. After addressing these issues, the newly-generated patient waveforms and spirometry images with the updated ventilation settings are classified as baseline images, and the baseline images, along with the ventilation settings, are time-stamped and stored in memory (e.g., in baseline images 114). After this step, the subsequent patient waveform and spirometry images are compared with the baseline waveform and spirometry images via the one or more comparison models. Image generation and comparison with the baseline images continues at the specific time interval until the patient ventilation support is removed (e.g., until the patient is weaned off the medical system 10).

Whenever changes are observed between the baseline images to new set of waveform and spirometry images, a notification/alert is sent to the anesthesiologist showing the deviations in the images with possible causes for the issue, when known. The anesthesiologist may act and addresses this issue by changing any ventilation settings, and the change in ventilation settings may be identified from the waveform and spirometry images. Now these waveforms and spirometry images become the new baseline and stored in the memory (e.g., in the baseline images 114). The previous baseline images and the patient waveform and spirometry images that triggered the change in the ventilation settings are moved to on-premise and/or remote data storage. This process continues and if patient spontaneous breathing efforts with an adequate tidal volume are observed (e.g., via the comparison models), then a weaning protocol may be executed (e.g., via the inference module 108) that includes a check of the patient monitor data for SpO2, blood pressure, etc., based on predetermined rules. If the patient monitor data passes the check, then an alert may be output to notify the anesthesiologist that the patient is ready for weaning. If the anesthesiologist accepts, then a further aspect of the weaning protocol may be executed that includes the output of audio commands (e.g., via speaker 126) that instruct the patient to open their eyes or perform another task that may be recorded via camera 124. The video from the camera may be evaluated via the comparison models to determine if the patient was able to follow the audio commands. Different patient face images and videos may be stored (e.g., in the on-premise and/or remote data storage) and used to train the comparison models to detect the changes in the patient face.

In current ventilation systems, the clinician is presented various patient/ventilation data such as pressure waveform, flow waveform, and volume waveform. The pressure, flow, and/or volume data may be combined to generate spirometry loops. Interpretation of the spirometry loops requires deep knowledge and good clinician expertise. Currently, no periodic baseline comparison data is available in ventilation systems to interpret these spirometry loops. Continuous monitoring of these waveform and spirometry images takes time and effort and inability to identify the changes in ventilation parameters may lead to compromised patient care. Thus, the embodiments disclosed herein of the continuous monitoring of the patient waveform and spirometry images is configured to help the clinicians to interpret the waveform and spirometry images, diagnose and identify the causes affecting the ventilation, like leak in the airway, obstruction in the airway, change in lung compliance, patient spontaneous breathing efforts, intrinsic PEEP (autoPEEP), etc. The methods disclosed herein monitor the ventilation throughout the procedure and alerts the clinicians whenever there is a change detected in the waveform and spirometry loops.

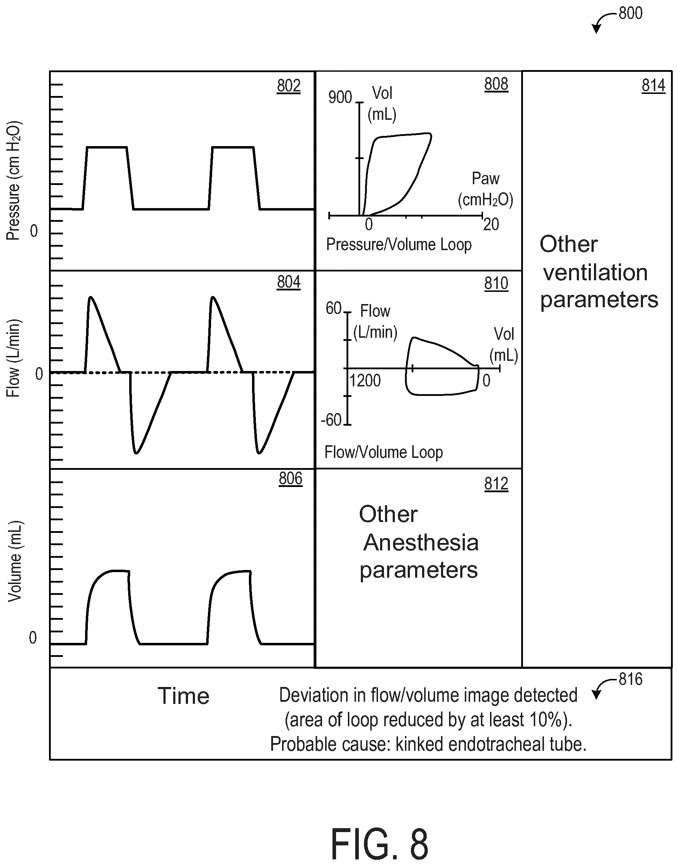

Turning briefly to FIG. 8, examples of patient ventilation parameter images that may be generated and evaluated as disclosed herein are shown. One or more sensors of the ventilation monitoring system 100 (e.g., one or more of the sensors 34) may be configured to measure patient airway pressure, airway flow, and airway volume over time. The output from the one or more sensors may be represented as time-series data, referred to as waveform images. FIG. 8 shows an example pressure waveform image 802 depicting airway pressure over time, an example flow waveform image 804 depicting airway flow over time, and an example volume waveform image 806 depicting airway volume over time. Each of the waveform images shown in FIG. 8 depict two respiration cycles, though it is to be appreciated that more or fewer respiration cycles may be depicted. The output from the one or more sensors may be represented as spirometry images, including an example pressure/volume spirometry image 808 that depicts airway volume as a function of airway pressure and an example flow/volume spirometry image 810 that depicts airway flow as a function of airway volume. The spirometry images shown in FIG. 8 depict pressure/volume and flow/volume over one respiration cycle.

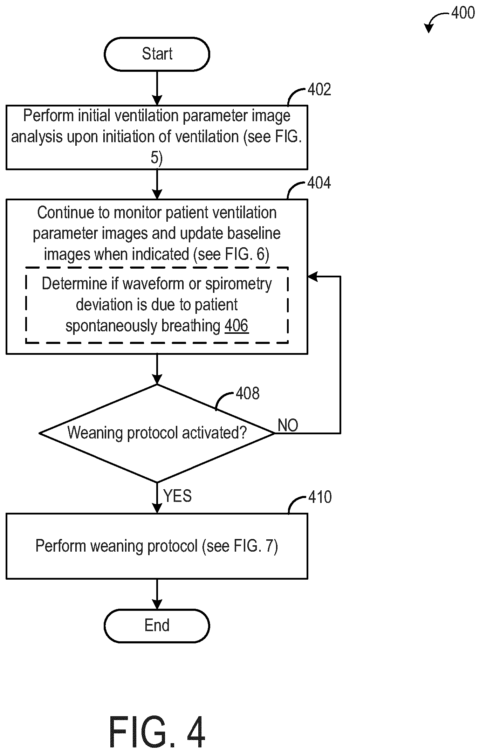

Returning to FIG. 3, the ventilation monitoring system 100 may be employed to monitor ventilation parameters, including the pressure, flow, and volume waveforms and the pressure/volume and flow/volume spirometry loops described above and shown in FIG. 8, of a patient during mechanical ventilation (e.g., when the patient is intubated and receiving respiratory support from the medical system 10), utilizing the one or more comparison models to identify deviations from expected ventilation parameters. The medical system 10 may include instructions stored in memory (e.g., memory 102) that may be executed by one or more processors (e.g., processing subsystem 58) to carry out the methods illustrated in FIGS. 4-7 and described below.

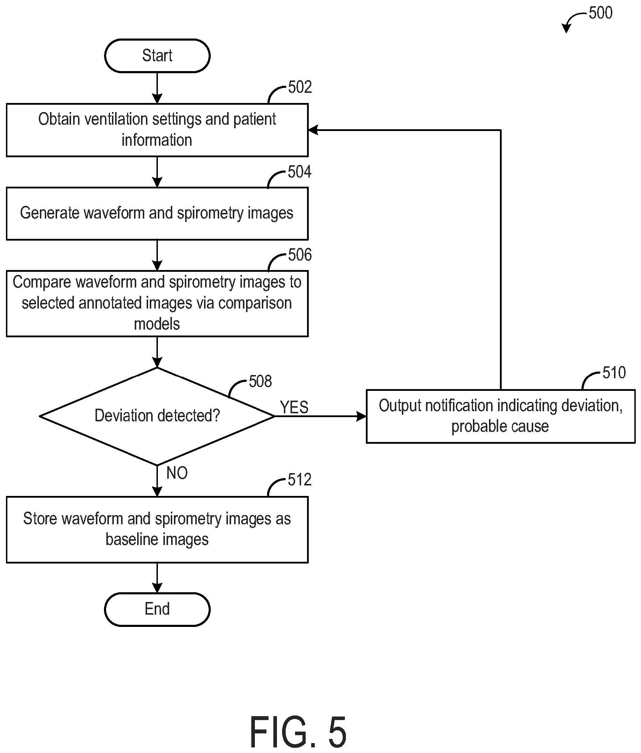

FIG. 4 shows a high-level method 400 for monitoring ventilation of a patient. Method 400 may be implemented with a ventilation monitoring system, such as the ventilation monitoring system 100 of FIG. 3, that includes a ventilation system, such as the medical system 10 of FIGS. 1 and 2. Method 400 may be carried out according to instructions stored in non-transitory memory and executed by one or more processors, such as memory and processor(s) of medical system 10. At 402, method 400 includes performing an initial ventilation parameter analysis upon initiation of ventilation. The initial ventilation parameter analysis is described in more detail below with respect to FIG. 5. Briefly, the initial ventilation parameter analysis may include generating initial patient ventilation parameter images (e.g., waveform images and spirometry images) from the output of one or more sensors of the ventilation system, as explained above and shown in FIG. 8, once mechanical ventilation has been initiated with initial ventilation settings. For example, the waveform images may include pressure, flow, and volume waveforms that depict airway pressure, flow, and volume, respectively, over time. The spirometry images may include pressure/volume and flow/volume spirometry loops that depict airway volume as a function of pressure and airway flow as a function of volume, respectively, where each spirometry loop depicts data for one respiration cycle. The initial patient ventilation parameter images may be compared to matched ground truth/annotated ventilation parameter images (via one or more comparison models) to determine if any deviations are present in the initial patient ventilation parameter images that suggest adjustments may be needed to the initial ventilation settings. If any deviations are detected, a notification may be output to the clinician(s) monitoring the patient so that adjustments may be made to the ventilation settings. If no deviations are detected, the initial patient ventilation parameter images may be classified as baseline images for the patient that reflect expected/baseline waveform and spirometry conditions/output. The baseline images may then be used to continue to monitor the patient.

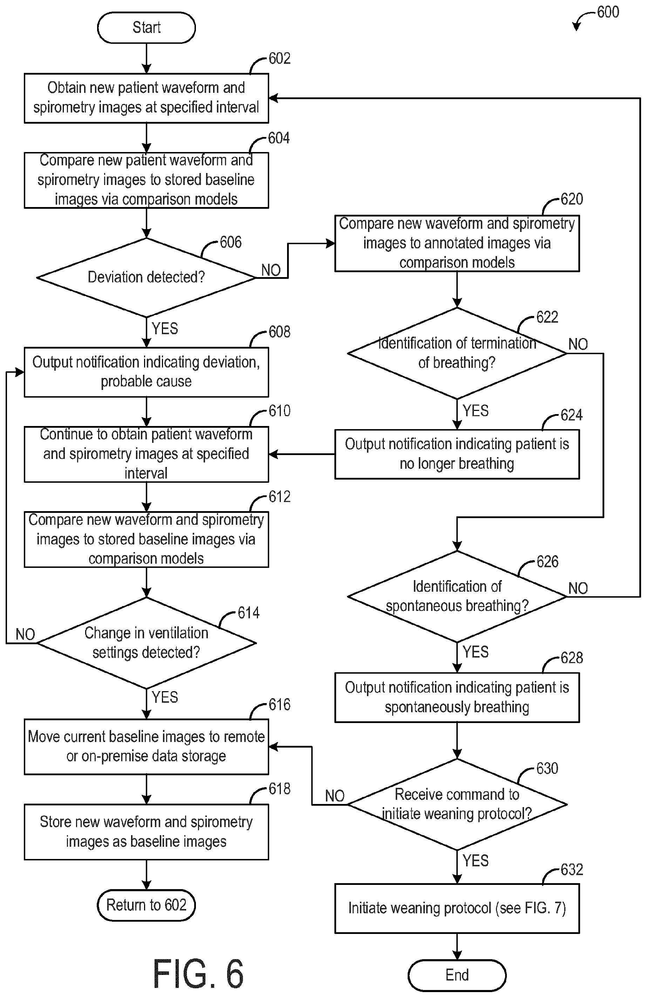

Accordingly, at 404, method 400 includes continuing to monitor subsequent patient ventilation images when indicated, which is explained in more detail below with respect to FIG. 6. After the initial waveform images and spirometry images have been evaluated as explained above, and baseline images for the patient are obtained, the ventilation system may continue to generate waveform images and spirometry images at a specified rate (e.g., once a minute, once every two minutes, etc.). Each set of waveform images and spirometry images may be compared to the baseline images via the one or more comparison models to determine if any (significant) deviations are detected. If any deviations are detected, a notification may be output to the clinician(s) monitoring the patient so that adjustments may be made to the ventilation settings.

The process of continuing to monitor the patient ventilation parameter images may include determining if any detected deviations (e.g., a waveform or spirometry deviation) are due to the patient breathing spontaneously, as indicated at 406. As will be explained in more detail below, as a patient's condition improves and/or as the procedure being performed on the patient reaches conclusion (and hence the need for mechanical ventilation diminishes), the patient may begin to breathe spontaneously, which may be observed in the waveform images and spirometry images. Detection of spontaneous breathing may trigger activation of a weaning protocol that may guide clinician decision-making around when to wean the patient from mechanical ventilation.

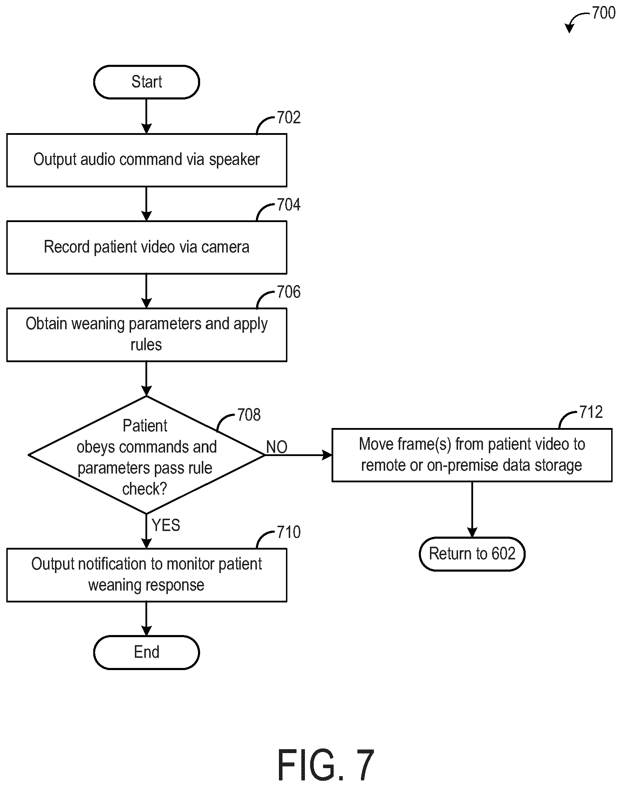

At 408, method 400 determines if the weaning protocol has been activated (e.g., due to detection of spontaneous breathing). If the weaning protocol has not been activated, method 400 proceeds back to 404 to continue to monitor the patient ventilation parameter images when indicated. If the weaning protocol has been activated, method 400 proceeds to 410 to perform the weaning protocol, which is described in more detail below with respect to FIG. 7. The weaning protocol may include rules-based checks of patient parameters (e.g., SpO2, PR, etc.) as well as automatic (e.g., via the ventilation system) determination of whether the patient is able to follow commands. If the weaning protocol indicates the patient may be ready for weaning, a notification may be output so that the clinician(s) monitoring the patient may wean the patient if desired. Method 400 then ends.

FIG. 5 is a flow chart illustrating a method 500 for performing an initial ventilation parameter analysis on a patient undergoing mechanical ventilation. Method 500 may be implemented with a ventilation monitoring system, such as the ventilation monitoring system 100 of FIG. 3, that includes a ventilation system, such as the medical system 10 of FIGS. 1 and 2. Method 500 may be carried out according to instructions stored in non-transitory memory and executed by one or more processors, such as memory and processor(s) of medical system 10. In some examples, method 500 may be carried out as part of method 400, such as at 402 of method 400.

At 502, method 500 includes obtaining ventilation system settings and patient information. The ventilation system settings may include, as non-limiting examples, whether mechanical ventilation is in pressure-targeted mode, volume control mode, or flow-targeted mode, set FiO2, set EtO2, set mechanical ventilation, set inspired anesthetic agent, and/or set expired anesthetic agent. The patient information may include patient age, gender, weight, and height. Additionally, the ventilation system settings and/or patient information may include measured ventilation parameters, such as airway resistance, peak inspiratory pressure, plateau pressure, PEEP, tidal volume, RR, inspiratory to expiratory time ratio (I:E), etc.

At 504, method 500 includes generating initial patient ventilation parameter images, such as one or more waveform images and/or one or more spirometry images. The first iteration of method 500 may include generation of initial waveform and spirometry images (e.g., the first waveform and spirometry images generated upon initiation of mechanical ventilation or once mechanical ventilation has been initiated and operating at steady-state). The waveform and spirometry images may be generated based on the output from the sensors of the ventilation system, such as sensors 34. For example, the sensors may output airway pressure, flow, and volume as time-series data. The waveform images may be generated (e.g., by the waveform processing module 106) by taking a snapshot of the time-series data. The waveform images may respectively capture pressure, flow, and volume over one or more respiration cycles. The spirometry images may be generated (e.g., by the waveform processing module 106) by plotting, for one respiration cycle, volume as a function of pressure and flow as a function of volume, and generating images of the plots. The spirometry images may be generated from the same sensor data captured in the waveform images, or from sensor data output within a threshold time of the sensor data captured in the waveform images (e.g., within one or two respiration cycles).

At 506, the (initial) waveform and spirometry images are compared to selected annotated images (e.g., ground truth images) via one or more comparison models. The selected annotated images may be obtained from an image store, such as annotated images 116 of FIG. 3, that stores a collection of annotated images. The annotated images in the image store may be waveform and spirometry images that capture expected or ground truth pressure, flow, and volume for a variety of patient demographics and ventilation system settings. The annotated images in the image store may be waveform and spirometry images obtained from ventilation systems while ventilating prior patients (with the prior patients spanning all age ranges, genders, and body types and the ventilation systems spanning various ventilation system settings). In some examples, the annotated images in the image store may include waveform and spirometry images generated with artificial lungs. The annotated images in the image store may be annotated with patient information, ventilation system settings, and expert-annotated indications of patient lung status (good or poor) and ventilation system status (no defects in system or one or more defects in the system, with the defects labeled).

The selected annotated images selected for comparison to the patient waveform and spirometry images may be selected based on the patient information and ventilation system settings obtained at 502, so that the selected annotated images are matched to the patient and ventilation system settings. For example, annotated images that demographically match the patient may be selected (e.g., if the patient is a male in their 50s that is 5'10" and weighs 170 pounds, the selected annotated images may be of prior patient(s) that are also male, in their 50s, and have similar height and weight). From the demographically-matched annotated images, the final selected images may be selected based on having the same or similar ventilation system settings (which may include the same or similar measured ventilation parameters). To the extent possible, the same types of ventilation parameter images may be selected, so that, if the patient ventilation parameter images include pressure, flow, and volume waveform images and pressure/volume and flow/volume spirometry images, the selected annotated/ground truth images are selected that include pressure, volume, and flow waveform images and pressure/volume and flow/volume spirometry images.

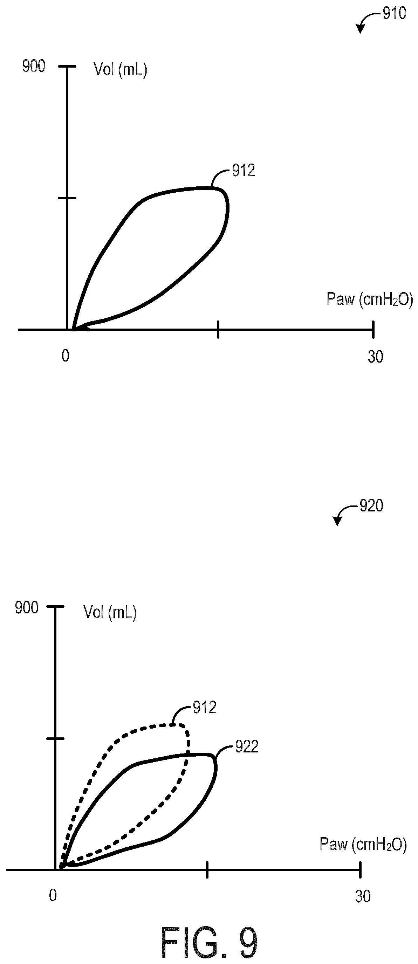

The one or more comparison models (such as the comparison models stored in the model repository 112) may be configured to detect various anomalies in the waveform and spirometry images, such as faulty deviations, hysteresis elongations, ineffective triggering, double triggering, open loop cycle, volume increases and reductions, and so forth. The one or more comparison models may detect these anomalies by comparing the patient waveform and spirometry images to the selected annotated waveform and spirometry images (e.g., comparing a patient pressure waveform image to an annotated/ground truth pressure waveform image; comparing a patient pressure/volume spirometry image to an annotated/ground truth pressure/volume spirometry image; etc.) and determining degrees of deviation/difference for one or more features. The one or more comparison models may extract only useful information from the various ventilation parameter images (e.g., the patient ventilation parameter images and the annotated/ground truth ventilation parameter images), such as the plotted data (e.g., curves, lines, loops) and the associated axes, using color segmentation, which may include converting the ventilation parameter images to respective binary masks (as explained in more detail below). Then, edge segmentation, adaptive thresholding, standard deviation determination, bit manipulation, edge detection, contour detection, density-based clustering, and/or combinations thereof may be applied via the one or more comparison models to the binary masks to detect one or more features of the patient ventilation parameter images and quantify the features, such as areas enclosed by curves/loops, the number of points of interception on x and/or y axes, angle and/or curvature of various lines, and so forth. Each feature may be compared to a corresponding feature of the annotated images to determine the differences/deviations in the one or more features and quantify the degree of deviation. Thresholding may be applied so that minor deviations are ignored, and the specific thresholds applied may depend on the feature. Additional details about the one or more comparison models and the one or more features analyzed via the one or more comparison models are presented below with respect to FIGS. 9-21. It is to be appreciated that the ventilation parameter images that are processed by the comparison models may be digital versions of the ventilation parameter images that include one or more numerical values for each pixel (e.g., a color value, brightness value, etc.), rather than the versions of the ventilation parameter images that are actually displayed and illustrated herein (e.g., where the pixel values have been converted to color/brightness). In some examples, in addition to analyzing the waveform and spirometry images, method 500 may include a comparison between other ventilation system parameters of the patient (e.g., Ppeak, Pplat, PEEP, FiO2, etc., as instantaneous or average values) and the corresponding ventilation system parameters of associated with the selected annotated images.

At 508, method 500 includes determining if any deviations are detected. The deviations may be differences in the at least one feature/parameter of the patient (e.g., the one or more features of the patient waveform and spirometry images) analyzed by the one or more comparison models relative to the at least one feature/parameter in the annotated images. A detected difference may only be identified as a deviation if the detected difference meets a condition relative to a threshold. For example, the area enclosed by a loop of a given patient spirometry image (e.g., a pressure/volume spirometry image) may be calculated via at least one of the one or more comparison models and compared to the area enclosed by the loop of the corresponding annotated spirometry image. If the area of the patient spirometry image differs from the area of the annotated spirometry image by more than a threshold (e.g., differs by at least 10%), the difference in area may be identified as a deviation. Conversely, if the area of the patient spirometry image differs from the area of the annotated spirometry image by less than the threshold (e.g., differs by less than 10%), the difference in area may not be identified as a deviation.

If a deviation is detected, method 500 proceeds to 510 to output a notification indicating the deviation and, when known, a probable cause of the deviation. The notification may be displayed on a display of the ventilation system (e.g., monitor 38) and/or one or more care provider devices (e.g., on care provider device 134 via graphical user interface 135). For example, if the patient flow/volume spirometry image has an enclosed area that is significantly smaller than the area of the selected annotated flow/volume spirometry image and the patient pressure/volume spirometry image has a significantly higher peak pressure than the selected annotated pressure/volume spirometry image, the probable cause of the reduction in the enclosed area may be a kinked endotracheal tube. The notification may include an indication that a deviation(s) was detected, a quantification of the deviation(s) (a percentage or actual deviation for each feature), and/or the probable cause.

When a deviation is detected and the notification output, the clinician(s) monitoring the patient may opt to take an action to address the deviation. In the example that the endotracheal tube may be kinked, the clinician may check the endotracheal tube and, if kinked, fix the endotracheal tube. In this way, in response to the notification being output, one or more ventilations system settings may be adjusted by the clinician. As such, method 500 may loop back to 502 to continue to obtain ventilation settings and patient information (as the ventilation settings may have changed), generate subsequent waveform and spirometry images (at a specified rate, such as every two minutes), and compare the subsequent waveform and spirometry images to selected annotated images via the one or more comparison models, to determine if the deviation persists or has been addressed. If the deviation persists, the notification may continue to be output (and the notification may be updated in some examples, if the deviation has changed owing to the changed ventilation settings).

If a deviation is not detected at 508 (whether no deviation is detected in the initial waveform and spirometry images or an initial deviation has been rectified and is no longer present), method 500 proceeds to 512 to store the current patient waveform and spirometry images as baseline images for the patient (e.g., in baseline images 114), which will be used as the basis for comparison for any further waveform and spirometry image analysis (explained below with respect to FIG. 6). It is to be appreciated that in some examples, when a notification is output indicating a deviation, the clinician may opt to ignore the notification or dismiss the notification. Thus, if a user input is received following output of a notification of a deviation (e.g., at 510) that dismisses the notification or otherwise indicates the clinician does not intend to adjust any ventilation settings as a result of the notification, the current patient waveform and spirometry images may be saved as the baseline images, even though they deviate from the selected annotated images. Because the selected annotated images are from other patients or based on simulated ventilation (with an artificial lung), the selected annotated images may not always reflect the expected or ideal for the actual patient. Thus, the user input dismissing the notification may act as an indication that the current patient waveform and spirometry images should be used as the baseline going forward. Similarly, if the notification is output at 510 but the deviation is not addressed after a threshold amount of time (e.g., five minutes, ten minutes), the current patient waveform and spirometry images may be saved as the baseline images. Method 500 then ends.

FIG. 6 is a flow chart illustrating a method 600 for performing continued ventilation parameter analysis on a patient undergoing mechanical ventilation. Method 600 may be implemented with a ventilation monitoring system, such as the ventilation monitoring system 100 of FIG. 3, that includes a ventilation system, such as the medical system 10 of FIGS. 1 and 2. Method 600 may be carried out according to instructions stored in non-transitory memory and executed by one or more processors, such as memory and processor(s) of medical system 10. In some examples, method 600 may be carried out as part of method 400, such as at 404 of method 400.

At 602, method 600 includes obtaining new patient ventilation parameter images (e.g., one or more waveform and/or spirometry images) at a specified interval, such as once a minute or once every two minutes. The new patient waveform and spirometry images may be obtained/generated as explained above with respect to FIGS. 4 and 5. At 604, the new patient waveform and spirometry images are compared to the stored baseline images for the patient via the one or more comparison models. The comparison may be performed as explained above with respect to FIG. 5, and as explained in more detail below with respect to FIGS. 9-21.

At 606, method 600 determines if any deviations are detected between the new patient waveform and spirometry images and the baseline images. As explained previously, the one or more comparison models may be deployed in order to process the new patient waveform and spirometry images and the baseline images to segment/isolate and quantify various features of the new patient waveform and spirometry images and the baseline images. Any changes in the analyzed features in the new patient waveform and spirometry images relative to the baseline images may be identified and compared to respective thresholds to determine if the change(s) are significant enough to be classified as a deviation.

If a deviation is detected, method 600 proceeds to 608 to output a notification indicating the deviation and a probable cause of the deviation (if known). The notification may be output on one or more displays, such as monitor 38 and/or displays of one or more care provider devices. At 610, new patient waveform and spirometry images continue to be obtained at the specified interval, and at 612, the new patient waveform and spirometry images are again compared to the baseline images for the patient via the comparison models.

At 614, method 600 determines if a change in ventilation settings has been detected. The change in ventilation settings may be detected based on the new patient waveform and spirometry images relative to the baseline images and/or the change in ventilation settings may be detected based on user input being received at the ventilation system actually changing the settings (e.g., changing gas flow parameters, anesthesia concentration, set FiO2, etc.). For example, when a deviation is detected but then the subsequent patient waveform and spirometry images revert back to being the same or similar to the baseline images, it may be inferred that the ventilation system settings have been changed.

If a change in ventilation settings is not detected, method 600 returns to 608 to continue to output the notification, obtain new patient waveform and spirometry images, and compare the new patient waveform and spirometry image to the baseline images. The process may repeat until a change in the ventilation settings is detected, or until a clinician dismisses the notification or a threshold amount of time has elapsed since the notification was originally output. If a change in ventilation settings is detected at 614, method 600 proceeds to move the current baseline images to remote or on-premise data storage (e.g., remote data storage 160 or on-premise data storage 150) at 616 and save the new patient waveform and spirometry images as the baseline images (e.g., in baseline images 114) at 618. When changes are made to the ventilation system settings, it may be assumed that the waveform and spirometry images that are generated after the change to the ventilation system settings reflect the new ideal/default parameters for the patient and the baseline images are updated accordingly. However, rather than discarding the prior baseline images, the prior baseline images may be saved in a new storage location, where the baseline images may be used to further refine the one or more comparison models. Method 600 then returns back to 602 to continue to monitor subsequent patient waveform and spirometry images.

Returning to 608, if a deviation is not detected between the new patient waveform and spirometry images and the baseline images, method 600 proceeds to 620 to compare the new patient waveform and spirometry images to selected annotated images via the one or more comparison models. The selected annotated images may be selected from the stored annotated images (e.g., from annotated images 116) based on the patient information and current ventilation system settings, similar to the selection of the annotated images described above with respect to FIG. 5. The selected annotated images may be used with the one or more comparison models to detect outlier conditions (rather than deviations from the patient's baseline). The outlier conditions may include termination of breathing and/or spontaneous breathing. Thus, at 622, method 600 may include determining if termination of breathing has been identified. Termination of breathing may be identified based on flat lines being present in the waveform images, for example. If termination of breathing has been identified, method 600 proceeds to 624 to output a notification indicating that the patient is no longer breathing, at which point method 600 may proceed to 610 to continue to obtain patient waveform and spirometry images and compare the patient waveform and spirometry images to the baseline images to determine if a change in ventilation settings is detected.

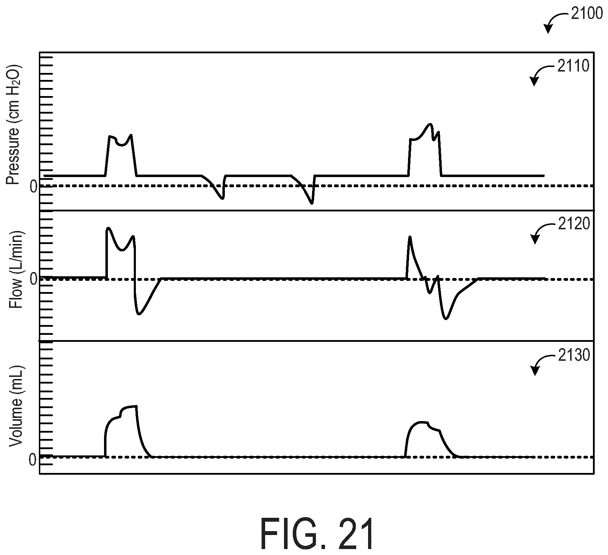

If termination of breathing is not identified, method 600 proceeds to 626 to determine if spontaneous breathing is identified based on the new patient waveform and spirometry images relative to the selected annotated images. For example, spontaneous breathing may be identified based on small negative deflections (e.g., drop in pressure) in the pressure waveform image, for example. Additional information about detecting spontaneous breathing is provided below with respect to FIG. 21. If spontaneous breathing is not identified, method 600 returns to 602 to continue to monitor subsequent patient waveform and spirometry images. If spontaneous breathing is identified, method 600 proceeds to 628 to output a notification (e.g., to a display such as monitor 38 and/or one or more care provider devices) indicating that the patient is spontaneously breathing. At 630, method 600 determines if a command to initiate a weaning protocol is received. For example, the notification that the patient is breathing may include a request to initiate the weaning protocol. Upon seeing the notification that the patient is spontaneously breathing, a clinician monitoring the patient may enter a user input that commands the ventilation system to initiate the weaning protocol (e.g., selection of a yes button or other user interface element). If a command to initiate the weaning protocol is not received (e.g., the clinician dismisses the notification or selects a no button), method 600 proceeds to 616. If the command to initiate the weaning protocol is received, method 600 proceeds to 632 to initiate the weaning protocol, which is explained in more detail below. In other examples, the ventilation system may automatically initiate the weaning protocol upon detecting the patient is spontaneously breathing. Method 600 then ends.

Thus, at the start of mechanical ventilation, a set of ventilation parameter images of a patient may be captured (e.g., pressure, flow, and volume waveform images and pressure/volume and flow/volume spirometry images) and compared to corresponding annotated/ground truth images selected based on patient and ventilation parameters (e.g., patient age, height, weight, and gender, and ventilation system settings) using at least one comparison model. This process may be used to "finalize" baseline images for the patient that reflect the current target ventilation waveforms and spirometry loops desired for the patient. For example, if the initial ventilation parameter images deviate from the annotated/ground truth images, the ventilation system settings may be adjusted. Once baseline images are captured, the baseline images may be compared to subsequent patient ventilation parameter images with the at least one comparison model. Any deviations from the baseline images may trigger output of a notification, so that a clinician can adjust ventilation system settings if indicated. During and/or after the procedure, the clinician can annotate any baseline or other ventilation parameter images and save in a database for future reference (e.g., along with the other annotated/ground truth images). As will be explained in more detail below, the one or more comparison models may characterize/quantify various features of the ventilation parameter images (both of the patient and the annotated/ground truth images), such as area enclosed by a curve, number of x and/or y intersection points, line angles, etc. The ventilation parameter images as well as quantified features may be saved locally (e.g., on the ventilation system) and sent to on-premise and/or remote data storage. In some examples, once an image has been characterized by the one or more comparison models, all the quantified features may be saved and used for any future comparisons. For example, a given baseline image (e.g., a pressure/volume spirometry loop) may be characterized by the one or more comparison models to determine, among other features, the area enclosed by a curve/loop in the image. Each time the given baseline image is compared to a subsequent patient ventilation image (e.g., a subsequent pressure/volume spirometry image), the quantified feature may be retrieved from memory for use in the comparison, rather than processing the baseline image through the one or more comparison models repeatedly, which may lower processing demands.

Further, the one or more comparison models disclosed herein may allow for characterization and quantification of various features of the ventilation parameter images, and the characterized/quantified features of reference ventilation parameter images (e.g., patient baseline images or annotated/ground truth images) may be compared to the characterized/quantified features of the current patient ventilation parameter images. By evaluating and comparing the ventilation parameter images (e.g., rather than evaluating only the sensor data), several benefits may be achieved. For example, some of the characterized features, such as area enclosed by a curve/loop of a spirometry image, are not identifiable in the sensor data used to generate the ventilation parameter images. Further, ventilation systems (e.g., ventilation and/or anesthesia machines) may not be configured to store the raw sensor data, but may be configured to store some ventilation parameter images (e.g., the last 5 images of each of the various waveform and spirometry images disclosed herein). Thus, by analyzing the ventilation parameter images, changes in patient condition may be detected using the resources already available on the ventilation system. Additionally, the one or more comparison models disclosed herein may be less resource-demanding (e.g., demand less memory and/or processing power) than other types of models (e.g., artificial intelligence models). Overall, the approach disclosed herein of evaluating ventilation parameter images may provide a mechanism to compare current patient ventilation parameters to expected ventilation parameters that takes of advantage of existing resources available on the ventilation system in a manner that is memory and processor efficient.

FIG. 7 is a flow chart illustrating a method 700 for performing a weaning protocol on a patient undergoing mechanical ventilation. Method 700 may be implemented with a ventilation monitoring system, such as the ventilation monitoring system 100 of FIG. 3, that includes a ventilation system, such as the medical system 10 of FIGS. 1 and 2. Method 700 may be carried out according to instructions stored in non-transitory memory and executed by one or more processors, such as memory and processor(s) of medical system 10. In some examples, method 700 may be carried out as part of method 400 and/or method 600, such as at 410 of method 400 and/or at 632 of method 600.

At 702, method 700 includes outputting an audio command via a speaker, such as speaker (e.g., speaker 126). The audio command may include instructions directed at the patient, such as instructions for the patient to open their eyes, close their eyes, and/or blink in a specific pattern (e.g., blink twice quickly). During output of the audio command and after output of the audio command, a patient video is recorded via a camera (e.g., camera 124), as indicated at 704. The patient video may include the patient in the field of view and may be a color video or a monochrome video. At 706, weaning parameters are obtained and weaning rules are applied to the weaning parameters to perform a rule-based check of the patient condition. The weaning parameters may include patient SpO2, blood pressure, PR, tidal volume, FiO2, EtCO2, and/or other parameters measured by the ventilation system and/or patient monitor. The weaning rules may include threshold ranges for each weaning parameter that indicate or suggest that the patient is not anesthetized and is able to breathe without assistance.

At 708, method 700 determines if the patient was able to obey the audio command and if the weaning parameters pass the rule-based check. The one or more comparison models may include an image model trained to identify whether the patient was able to obey the audio command. For example, the image model may be a neural network trained to identify whether the patient's eyes are open or closed in each image frame of the patient video, and the method may determine that the patient was able to obey the audio command based on whether (and when) the patient's eyes were open and closed in the patient video relative to when the audio command was issued. The weaning parameters may pass the rule-based check if each weaning parameter is within the specified threshold range for that parameter. For example, the rule-based check may include determining if the patient is making spontaneous breathing efforts for more than a threshold number of consecutive breaths (e.g., five), if SpO2 is more than a threshold value (e.g., 96%), and if EtCO2 is within a threshold range (e.g., 35-45 mm Hg). If all the rules/parameters are met, the weaning parameters may pass the rule-based check.

If the patient was able to obey the audio command and the weaning parameters did pass the rule-based check, method 700 proceeds to 710 to output a notification (e.g., for display on monitor 38 and/or one or more care provider devices) to monitor the patient weaning response. The notification may alert the clinician(s) monitoring the patient that the patient is ready for weaning and the clinician(s) may initiate the weaning and extubation process, at which point method 700 may end. If the patient was not able to obey the audio command, or if the weaning parameters did not pass the rule-based check, method 700 proceeds to 712 to move the frame(s) from the patient video to the remote or on-premise storage for further refinement of the image model. Method 700 then proceeds to 602 to continue monitoring the patient waveform and spirometry images, as the patient did not display signs that the patient was ready for weaning.

FIG. 8 shows an example graphical user interface 800 that may be displayed on a monitor of a ventilation system, such as monitor 38, during execution of the methods described above. The graphical user interface 800 may display the pressure waveform image 802, flow waveform image 804, volume waveform image 806, pressure/volume spirometry image 808, and flow/volume spirometry image 810. The graphical user interface 800 may further display other anesthesia parameters 812 (such as inspired anesthetic agent concentration and expired anesthetic agent concentration) and other ventilation parameters 814 (e.g., PEEP, tidal volume). The graphical user interface 800 may also display the notifications generated and output during execution of the methods described above. For example, a notification 816 may be displayed indicating that a deviation in a flow/volume spirometry image has been detected, with a probable cause of a kinked endotracheal tube.

FIG. 9 shows a first set of spirometry images that may be evaluated to determine a change in patient ventilation status. The first set of spirometry images may be generated with the medical system 10 during execution of one or more of the methods described above with respect to FIGS. 4-7 and analyzed via the one or more comparison models (e.g., the comparison models of the model repository of FIG. 3). The first set of spirometry images includes a first spirometry image 910 and a second spirometry image 920. The first spirometry image 910 is a pressure/volume spirometry image that depicts patient airway volume as a function of patient airway pressure for one respiration cycle. Because one respiration cycle includes both inhalation and exhalation, the first spirometry image 910 may include a first loop 912 of patient pressure and volume. Features of the first loop 912 may be evaluated by the one or more comparison models to establish a baseline for the patient, as the first spirometry image 910 may be an initial image that captures an initial status of the patient.

The second spirometry image 920 is also a pressure/volume spirometry image and includes a second loop 922 of patient pressure and volume, captured after the first spirometry image 910. Features of the second loop 922 may be evaluated by the one or more comparison models and compared to the features of the first loop 912 to determine if the patient status has changed relative to the initial/baseline status. For illustration, the first loop 912 is also included in the second spirometry image 920 to illustrate the change in patient status.

In order to evaluate the features of the first loop 912 and the second loop 922, the loops may be extracted from the spirometry images using a first comparison model. The first comparison model may be configured to perform color segmentation by grouping pixels of the spirometry image that have a common characteristic, such as color. The first comparison model, in order to perform the color segmentation, may convert the image (e.g., the second spirometry image 920) to a suitable color space, such as hue, saturation, and value (HSV), and convert the image (in the selected color space) to a binary mask where pixels in a selected color range are set to a value of 1 and all other pixels are set to a value of 0, for example. The color segmentation may thus extract the loop or waveform and the axes. After extraction, any spaces (e.g., where portions of the loop or axes were unevenly extracted) may be filled using binary closing (e.g., performing dilation followed by erosion). The final extracted image may be used for downstream analysis via additional comparison model(s), such as determining area, intersection points, etc., as explained in more detail below. The first comparison model may have tunable parameters that include HSV selection and the binary mask (e.g., the color range used to set the values of each pixel of the binary mask, which may depend on the colors present in the ventilation parameter image). By converting the ventilation parameter images to respective binary masks and performing downstream computer vision/image processing techniques on the binary masks via the one or more comparison models, memory demands may be reduced by storing the binary mask instead of the full image, and the processing demands of the comparison models in performing the computer vision/image processing techniques may be reduced by not having to process full images.

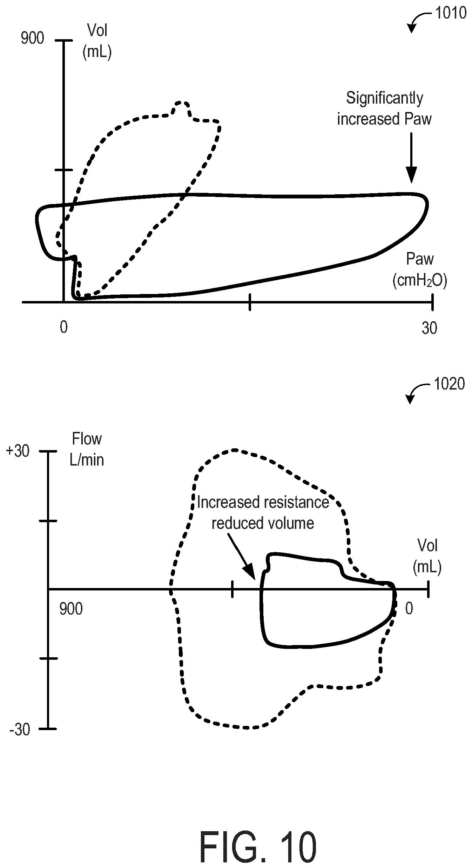

FIG. 10 shows a second set of spirometry images that may be evaluated to determine a change in patient ventilation status. The second set of spirometry images may be generated with the medical system 10 during execution of one or more of the methods described above with respect to FIGS. 4-7 and analyzed via the one or more comparison models (e.g., the comparison models of the model repository of FIG. 3). The second set of spirometry images includes a first spirometry image 1010 and a second spirometry image 1020. Similar to the second spirometry image 920 of FIG. 9, each of the first spirometry image 1010 and the second spirometry image 1020 includes two loops, a prior (e.g., baseline) loop and a current loop. The first spirometry image 1010 is a pressure/volume spirometry image and the second spirometry image 1020 is a volume/flow spirometry image. Each of the first spirometry image 1010 and the second spirometry image 1020 illustrate features indicative of a kinked endotracheal tube. After color segmentation via the first comparison model, a second comparison model may determine the x max point of each loop in the first spirometry image 1010, which is the highest airway pressure of the respiration cycle. As appreciated by the first spirometry image 1010, the current loop (shown as the solid line) has an x max point that is significantly higher than the x max point of the prior loop (shown as the dashed line). The increased maximum airway pressure is indicative of a kinked endotracheal tube, as the kink provides resistance to the exhaled gas. Similarly, after color segmentation via the first comparison model, a third comparison model may determine the area enclosed by each loop of the second spirometry image 1020. As appreciated by the second spirometry image 1020, the current loop (solid) has a significantly smaller area than the area of the prior loop (dashed). The increased resistance of the kinked endotracheal tube reduces the volume of inhaled and exhaled gas.

The third comparison model may determine the area enclosed by a loop by employing color segmentation (which may instead be determined by the first comparison model), edge segmentation, contour detection, and drawing. The tunable parameters of the third comparison model include HSV selection and the binary mask (for color segmentation), threshold and mode in edge detection, and mode and method for contour detection. The third comparison model may use the binary mask as a cleaning tool (e.g., after edge segmentation, contour detection, and drawing, the binary mask may be applied to remove any features outside of the resultant curve/loop). After color segmentation, the third comparison model may find out the contours of the loop (e.g., in the segmented image) using edge segmentation and finally a masked and highlighted image is formed to calculate only the area enclosed by the curve/loop. In some examples, the loop may be segmented into a first portion above the x axis, for example, and/or a second portion below the x axis and the area of one or both portions calculated. This may include detecting the contours, then, to determine the area of the portion above the x axis, considering only the points to form the contour above the x axis, and finally creating a mask to highlight only the area of the curve above the x axis. In some examples, the ratio of the upper portion (e.g., above the x axis) to the overall area may be determined. The overall area can be calculated before masking only the certain points.

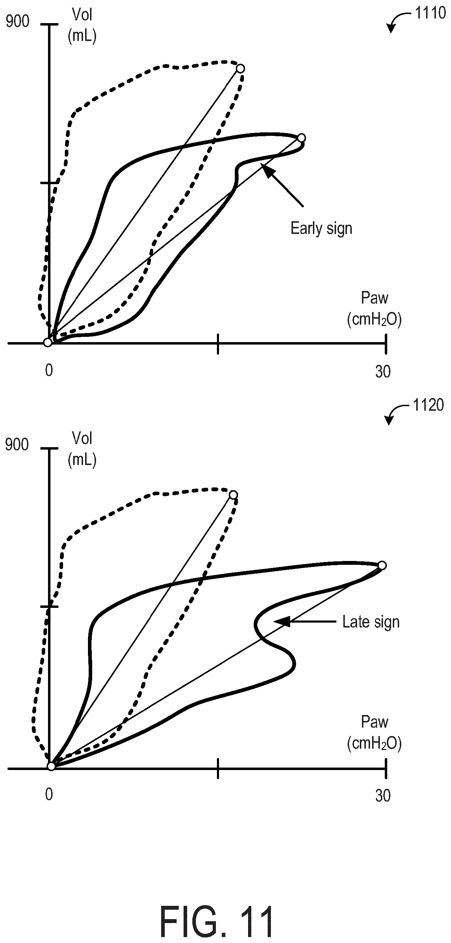

FIG. 11 shows a third set of spirometry images that may be evaluated to determine a change in patient ventilation status. The third set of spirometry images may be generated with the medical system 10 during execution of one or more of the methods described above with respect to FIGS. 4-7 and analyzed via the one or more comparison models (e.g., the comparison models of the model repository of FIG. 3). The third set of spirometry images includes a first spirometry image 1110 and a second spirometry image 1120. Similar to the second spirometry image 920 of FIG. 9, each of the first spirometry image 1110 and the second spirometry image 1120 includes two loops, a prior (e.g., baseline) loop (shown in dashed) and a current loop (shown in solid). The first spirometry image 1110 and the second spirometry image 1120 are both a pressure/volume spirometry image. Each of the first spirometry image 1110 and the second spirometry image 1120 illustrate features indicative of the patient breathing against the ventilator (e.g., lack of relaxation).

After color segmentation via the first comparison model and determination of the x max point via the second comparison model, a fourth comparison model may determine the angle between the line connecting the x max point and a bottom right point for each loop. The first spirometry image 1110 shows, for the current loop, an early sign of the patient breathing against the ventilator while the second spirometry image 1120 shows, for the current loop, a late sign of the patient breathing against the ventilator.

The fourth comparison model may employ color segmentation, edge segmentation, and contour detection, with tunable parameters of HSV selection and the binary mask (e.g., for color segmentation), threshold and mode in edge detection, and mode and method for contour detection. The fourth comparison model may utilize a binary mask as a cleaning tool. The fourth comparison model identifies the highest point on the x axis and draws a line to a fixed point at the bottom right corner. The deviation in the angle formed by the line with the positive direction of the x axis is calculated. Similarly, a threshold can be set to a minimum degree to find out the deviation from the baseline (e.g., prior) image.

For example, lines have been placed on the first and second spirometry images as described above to show that the angle of the line for each current loop is significantly different than the angle for the corresponding line for each of the prior loops (e.g., showing that at the x max point/highest airway pressure, the airway pressure is higher for the current loops than the prior loops and also the airway volume at the highest airway pressure is lower for the current loops than the prior loops). The patient breathing against the ventilator may be evaluated by checking the angle of the line. If the angle of the line is not within a threshold, a deviation is detected. If the angle is within the threshold, the x max and y max values are determined. If the x max and y max values are not within a threshold, a deviation is detected. Further, from the origin, if the line that is placed as described above intersects the curve at more than two locations and a small area is covered on the left side of the line, a deviation is detected. In the example shown in FIG. 11, the inward curves (where the early sign and late sign are noted) result in the lines intersecting the curves at more than two locations.

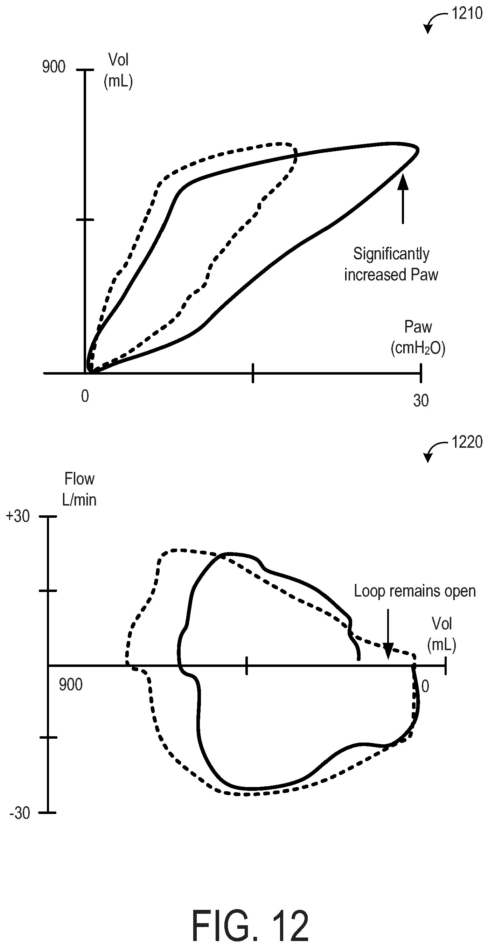

FIG. 12 shows a fourth set of spirometry images that may be evaluated to determine a change in patient ventilation status. The fourth set of spirometry images may be generated with the medical system 10 during execution of one or more of the methods described above with respect to FIGS. 4-7 and analyzed via the one or more comparison models (e.g., the comparison models of the model repository of FIG. 3). The fourth set of spirometry images includes a first spirometry image 1210 and a second spirometry image 1220. Similar to the second spirometry image 920 of FIG. 9, each of the first spirometry image 1210 and the second spirometry image 1220 includes two loops, a prior (e.g., baseline) loop (in dashed) and a current loop (in solid). The first spirometry image 1210 is a pressure/volume spirometry image and the second spirometry image 1220 is a volume/flow spirometry image. The first spirometry image 1210 illustrates features indicative of unintentional one-lung intubation and the second spirometry image 1220 illustrates features indicative of a leak in the ventilation system, in particular a leak in a cuff or laryngeal mask.

The unintentional one-lung intubation may be detected based on a significantly increased pressure, which may be detected by identifying the x max point and drawing a line from the x max point to a fixed point at the bottom right corner (as explained above). The deviation in the angle formed by the line with the positive x direction is calculated. A threshold may be set to a minimum degree to find out the deviation from the prior image, using the basics of color segmentation. The x max and y max values may also be compared.

A leak in the ventilation system may be detected based on the output of a fifth comparison model and/or a sixth comparison model, each of which may be configured to generate output that indicates if a spirometry loop (e.g., a volume/flow loop) is open or closed. After color segmentation via the first comparison model, the fifth comparison model may determine the points of intersection of the loops in a spirometry image, such as the second spirometry image 1220. The fifth comparison model may be configured to carry out color segmentation (or alternatively, the color segmentation may be performed by the first comparison model), edge segmentation, contour detection, and DBSCAN. The tunable parameters of the fifth comparison model include HSV selection and the binary mask (e.g., for color segmentation), threshold and mode in edge detection, mode and method for contour detection, EPS and minimum number of points in a cluster, and reference height of the x axis. The fifth comparison model may use a binary mask as a cleaning tool.

Thus, the fifth comparison model, after extracting the colored parts of the image, determines the points of intersection (POI) of the loops with the x axis. The POI in the prior loop of the second spirometry image 1220 may be positioned differently and/or be more or fewer in number than the POI of the current loop, which may indicate the leak due to the current loop being an open loop. Additionally or alternatively, the sixth comparison model, after color segmentation, may approximate the perimeter of the loop to a nearby polygon. If the polygon is open, a leak may be indicated. For example, a line may be drawn starting, for example, at a top of the loop that follows the contours of the loop using curve convexity. The starting point and ending point of the line may be evaluated to determine if the polygon is open or closed based on a distance between the starting point and the ending point.

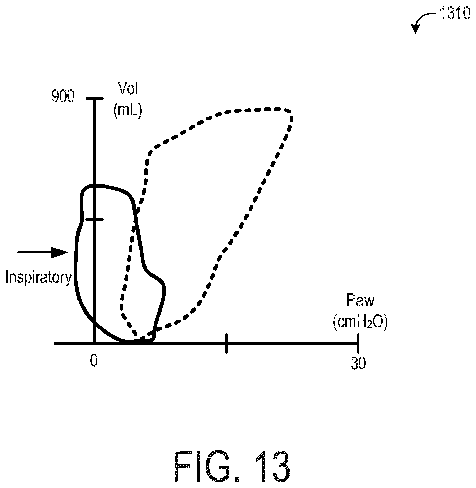

FIG. 13 shows another spirometry image, specifically a spirometry image 1310, that may be evaluated to determine a change in patient ventilation status. The spirometry image 1310 may be generated with the medical system 10 during execution of one or more of the methods described above with respect to FIGS. 4-7 and analyzed via the one or more comparison models (e.g., the comparison models of the model repository of FIG. 3). The spirometry image 1310 includes two loops, a prior (e.g., baseline) loop and a current loop. The spirometry image 1310 is a pressure/volume spirometry image. The spirometry image 1310 illustrates features indicative of spontaneous breathing (e.g., negative airway pressure during a portion of the respiration cycle), specifically the prior/baseline loop is entirely on the positive side of the plot while the current loop includes a portion on the negative side of the plot.

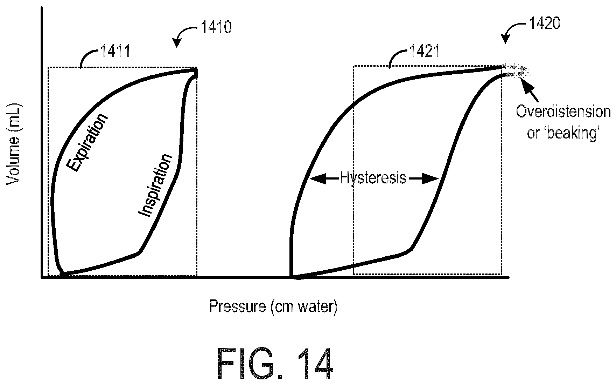

FIG. 14 shows a fifth set of spirometry images that may be evaluated to determine a change in patient ventilation status. The fifth set of spirometry images may be generated with the medical system 10 during execution of one or more of the methods described above with respect to FIGS. 4-7 and analyzed via the one or more comparison models (e.g., the comparison models of the model repository of FIG. 3). The fifth set of spirometry images includes a first spirometry image 1410 and a second spirometry image 1420. The first spirometry image 1410 may include a prior (e.g., baseline) loop and the second spirometry image 1420 may include a current loop. The first spirometry image 1410 and the second spirometry image 1420 are both a pressure/volume spirometry image. The first spirometry image 1410 and the second spirometry image 1420 illustrate features that may be evaluated by a seventh comparison model configured to identify over-distention or "beaking" (e.g., beak formation) of a spirometry loop (as shown by the marked points on the loop of the second spirometry image 1420).

The seventh comparison model may employ thresholding and contour detection and may have tunable parameters that include threshold values and mode, contour retrieval mode and method, and length and width of a rectangle. The seventh comparison model applies a threshold for the curvature of the loop as a piece of a rectangle with a length minimum of 60 pixels and a height minimum of 15 pixels. The reference point is taken as the point with the x_max. The size of the rectangle may be determined by the baseline/reference image. For example, the rectangle may be drawn based on the x max and y max points of the baseline/reference image, as shown by the rectangle 1411. A rectangle 1421 of the same size may be placed on the second spirometry image 1420. The number of points outside the rectangle may be counted. A greater the number of points indicates that there is an elongation to the hysteresis, while a normal loop does not have as much of the hysteresis. The number of points in the region can be utilized to find out the extension. Hysteresis extension indicates that too much pressure is applied to the lung and the lung is expanded beyond an acceptable threshold, which may cause barotrauma.

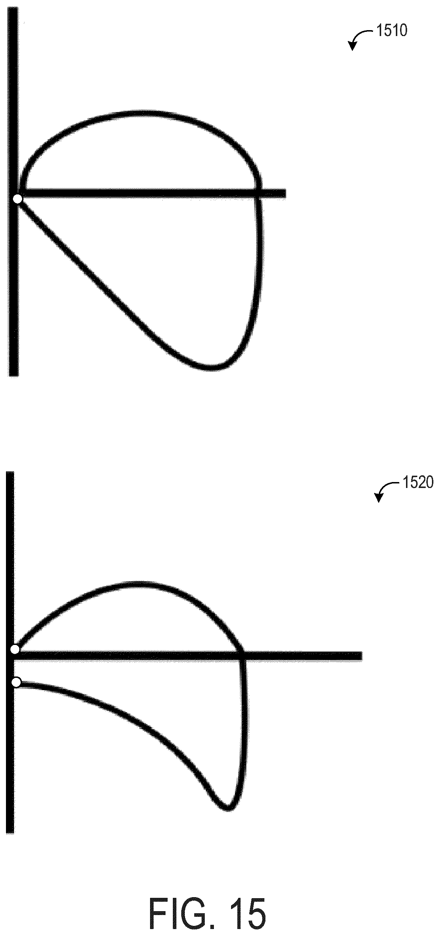

FIG. 15 shows two example spirometry loops, in this example volume/flow loops, that have been evaluated by an eighth comparison model to determine the number of intersection points on the y axis. The eighth comparison model may employ thresholding, DBSCAN, and contour detection. The tunable parameters of the eighth comparison model may include threshold values and mode, contour retrieval mode and method, reference value of the x axis and y axis (e.g., the location of the y axis) and the acceptable points value, EPS and minimum number of points in a cluster.

The eighth comparison model is configured to find the points of intersection with the y axis, and the parameters to be adjusted are the EPS in DBSCAN. The eighth comparison model selects the approximate distance of the y axis, calculated by finding out the point which is at the minimum value of the x axis (e.g., the x_min). For volume/flow loops, more than one POI on the y axis, along with a change in the slope of the expiratory curve, indicates air trapping due to potential obstruction in the airway.

For example, the first spirometry loop 1510 in FIG. 15 includes one point of intersection on the y axis, while the second spirometry loop 1520 includes two points of intersection with the y axis and an increased curvature of the expiratory curve, which is detected with a ninth comparison model, explained below.

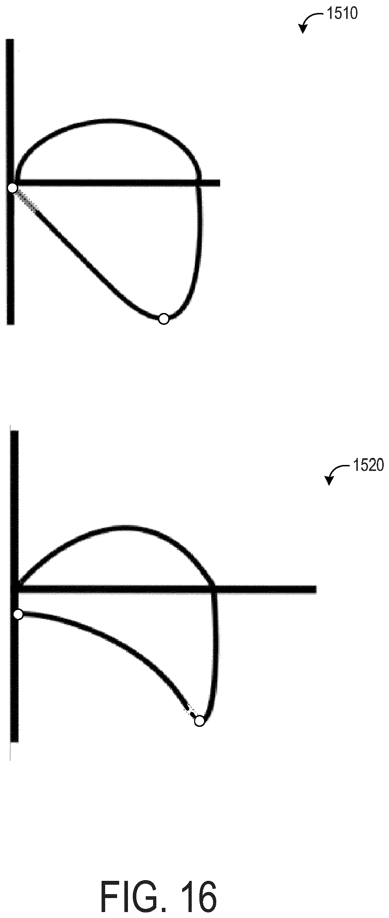

FIG. 16 shows the spirometry loops of FIG. 15 after evaluation by a ninth comparison model configured to determine the number of points along a line of a loop joining the lowest point of the loop and the y axis. The ninth comparison model may employ thresholding, DBSCAN, and contour detection, with tunable parameters that include threshold values and mode, contour retrieval mode and method, reference value of the y axis (location of y axis) and the acceptable points value, EPS and minimum number of points in a cluster. The ninth comparison model may compare the lowest point on the curve in the y-axis to the lowest point of intersection of the curve on the y axis. The number of points between the line may define the radius of curvature of the line/curve. The greater the points on the curve, the straighter the line.

For example, the lowest points of the loops and the intersections with the y axis are marked on the first spirometry loop 1510 and the second spirometry loop 1520 in FIG. 16. The points of each loop that lie on/intersect with the lines drawn between the lowest points and y intercept are shown by the gray points on the loops. The first spirometry loop 1510 has more points marked than the second spirometry image, indicating the portion of the first spirometry loop between the lowest point of the curve (the y_min) and the y intercept is straighter than the equivalent portion of the second spirometry loop.

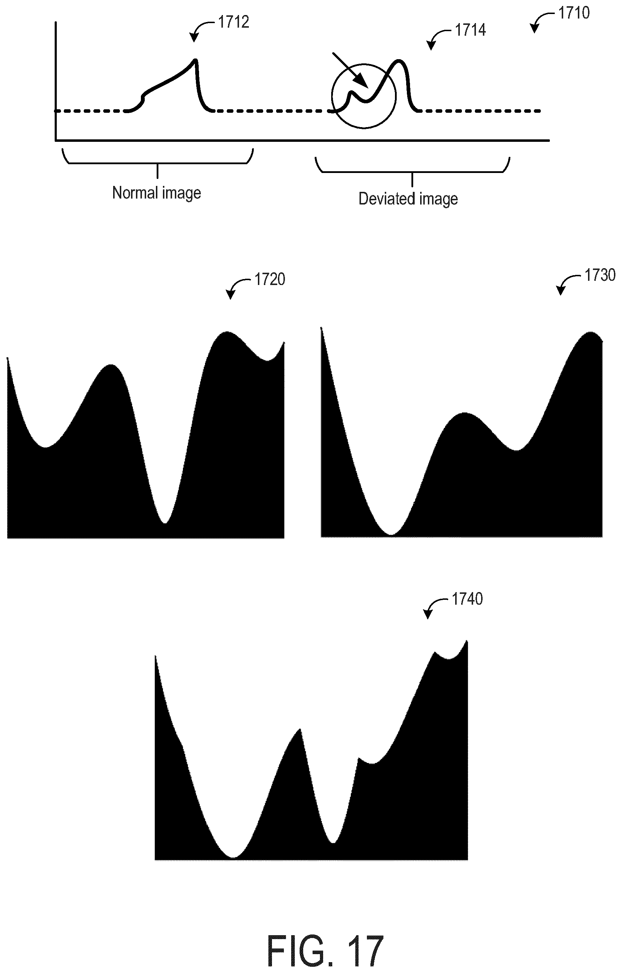

FIG. 17 shows a first set of waveform images 1710 that may be evaluated to determine a change in patient ventilation status. FIG. 17 also shows intermediate images generated by a tenth comparison model during evaluation of the first set of waveform images 1710. The first set of waveform images 1710 may be generated with the medical system 10 during execution of one or more of the methods described above with respect to FIGS. 4-7 and analyzed via the one or more comparison models (e.g., the comparison models of the model repository of FIG. 3).

The first set of waveform images 1710 includes a normal/ground truth image 1712 (which may be a baseline image in some examples) and an image showing a deviation (referred to as a deviated image 1714). The tenth comparison model may identify a common area between the curves of the normal image 1712 and the deviated image 1714. The tenth comparison model may employ adaptive thresholding and bit manipulation on pixels of the images, with tunable parameters that include threshold values and mode, and cropping image values. The tenth comparison model identifies the overlapping area between the two curves (herein, the curve of the normal image 1712 and the curve of the deviated image 1714). Identifying the overlapping area includes Or'ing two images formed from the normal image 1712 and the deviated image 1714, herein first image 1720 and second image 1730, respectively, to form a final image 1740 and the area is found out by counting the number of black pixels in the final image 1740. To generate the two images (e.g., the first image 1720 and the second image 1730), Matplotlib may be applied to the normal image 1712 and the deviated image 1714 and then the output is converted to images. Further, bitwise_or is used to generate the final image 1740.

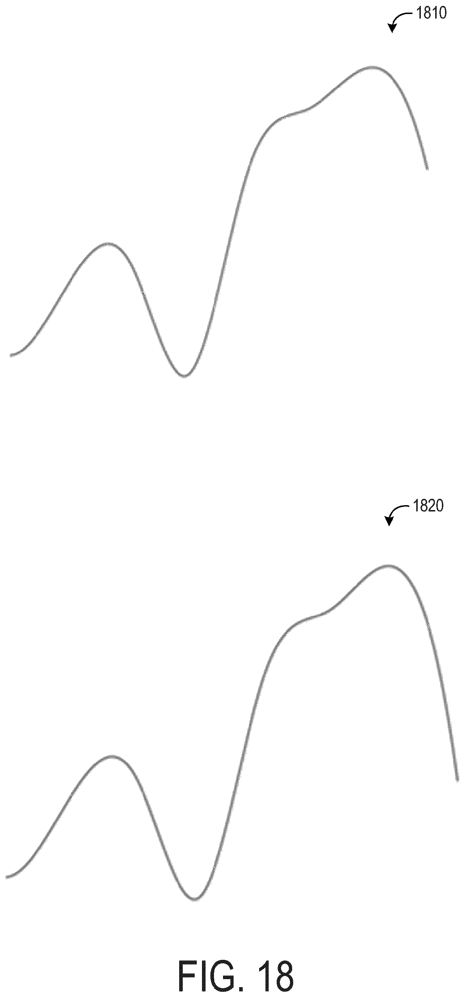

FIG. 18 shows the output of an eleventh comparison model that may be applied to ventilation parameter images, such as the normal image 1712 and deviated image 1714 of FIG. 17. The eleventh comparison model may determine a point-to-point deviation and standard deviation between two curves. The eleventh comparison model may employ adaptive thresholding, contour detection, and standard deviation, with tunable parameters of threshold values and mode, cropping image values, and contour retrieval mode and method.

The eleventh comparison model uses the point-to-point standard deviation along with the overall standard deviation, where two curves, such as first curve 1810 and second curve 1820, are compared point-wise and then the pixel distance is calculated. The two curves are ensured to be of equal length by zero padding the arrays. A deviation may be detected by setting a tolerable standard deviation value, and any value beyond that can be treated as a deviation. For example, the point-wise deviation of the second curve 1820 may be determined relative to the first curve 1810 and an overall standard deviation of deviation between the two curves may be determined.

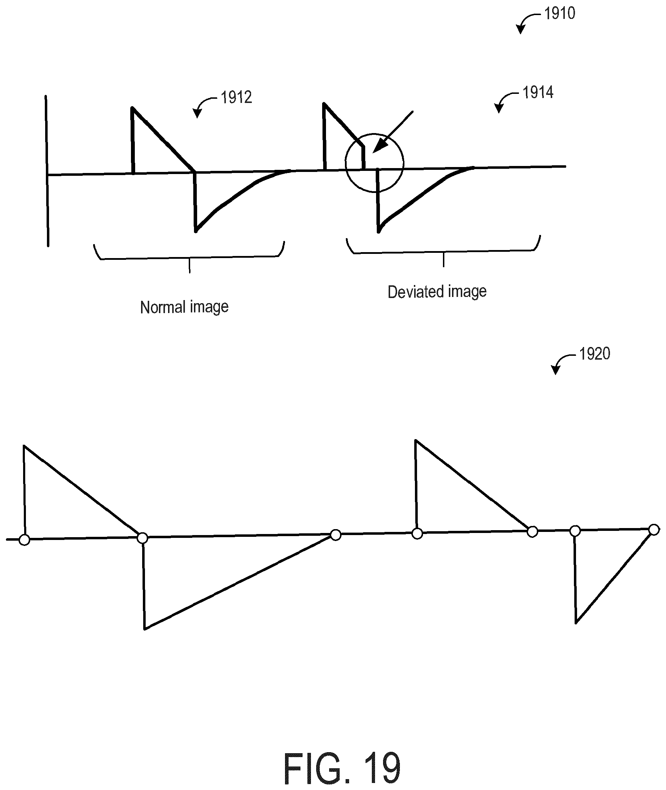

FIG. 19 shows a second set of waveform images 1910 that may be evaluated to determine a change in patient ventilation status. FIG. 19 also shows output 1920 of a twelfth comparison model during evaluation of the second set of waveform images 1910. The second set of waveform images 1910 may be generated with the medical system 10 during execution of one or more of the methods described above with respect to FIGS. 4-7 and analyzed via the one or more comparison models (e.g., the comparison models of the model repository of FIG. 3). In the example shown, each waveform of the set of waveform images 1910 is a flow waveform.

The second set of waveform images 1910 includes a normal/ground truth image 1912 (which may be a baseline image in some examples) and an image showing a deviation (referred to as a deviated image 1914). The twelfth comparison model may identify the number of points of intersection with the x-axis. The twelfth comparison model may employ edge detection, contour detection, and density-based clustering (DBSCAN), with tunable parameters that include threshold values and mode, the reference height of the x axis in the image, DBSCAN parameters (EPS) and minimum number of elements in a cluster, and distance of points to be considered from the reference x axis. The twelfth comparison model may identify and count the number of intersection points of the waveform on the x axis (e.g., as shown by the output 1920, with the intersection points shown by the dots placed on the x axis). If the number of intersection points is even, then the waveform may be identified as having a deviation (specifically a gap between loops, as shown by the arrow for the waveform of the deviated image 1914); however, if the number of intersection points is odd, then the waveform may be identified as normal (as shown for the waveform of normal/ground truth image 1912). The increase in number of intersection points signifies that a gap may be present in the patient's respiration cycle owing to no gas flow during inspiration. Each cycle should ideally have 3 POI according to the input.

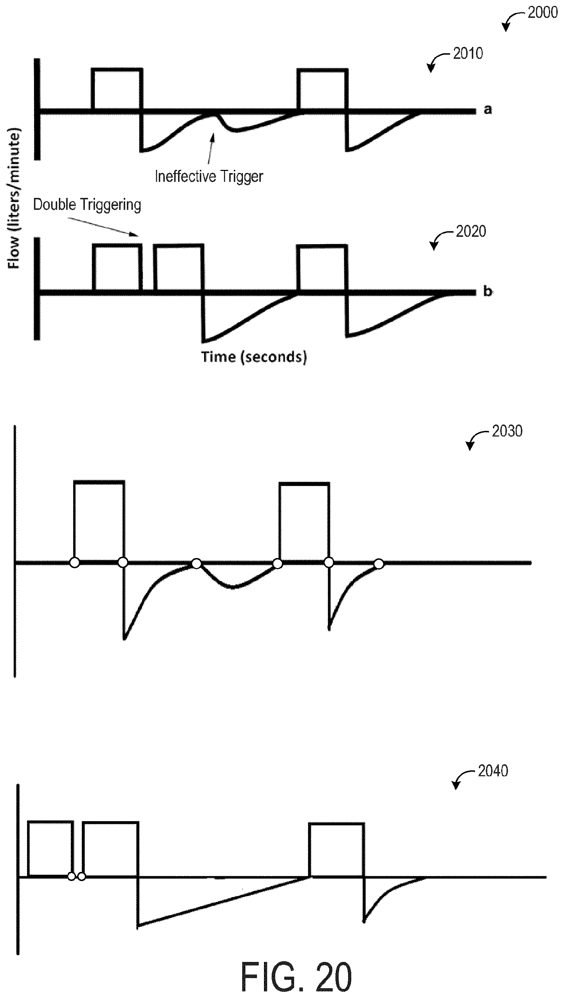

FIG. 20 shows a third set of waveform images 2000 that may be evaluated to determine a change in patient ventilation status. FIG. 20 also shows first output 2030 and second output 2040 of the twelfth comparison model during evaluation of the third set of waveform images 2000. The third set of waveform images 2000 may be generated with the medical system 10 during execution of one or more of the methods described above with respect to FIGS. 4-7 and analyzed via the one or more comparison models (e.g., the comparison models of the model repository of FIG. 3). In the example shown, each waveform of the third set of waveform images 2000 is a flow waveform showing two consecutive respiration cycles.

The third set of waveform images 2000 illustrate examples of patient-ventilator asynchrony that can be seen on the flow versus time waveform as a deflection from the baseline expiratory flow with either a minimal decrease in airway pressure or no change at all, and no breath delivered. For example, a first waveform image 2010 shows an ineffective trigger, where the expiratory flow during the first illustrated respiration cycle is extended and a subsequent inspiratory phase is skipped. The ineffective trigger may be detected based on the number of points of intersection with the x axis during each respiration cycle (e.g., more than three intersection points may be indicative of an ineffective trigger), as shown by the intersection points of the first output 2030, as well as process one cycle section and the slope pattern of the curve. In another example, ineffective triggering may be identified via determining the distance between peaks. For example, after color segmentation, the contours are detected and finally the points that lie on a reference line are masked. Clustering is performed to find only one point that intersects the reference line. The anomaly can be detected by finding the distance between the two consecutive points. If it is greater than a threshold number of pixels, then there is ineffective triggering.

For example, the ineffective trigger may be identified by a process that includes:
Step 1: Find the intersection points in X-axis.
Step 2: For the first respiration cycle, find the first 3 POIs and find the Y-max of the first two intersection points.
Step 3: Check the next intersection point and find its Y-value and compare with Y-max. If it's less than Y-max, then consider as expiratory flow is extended and confirm as an ineffective trigger.
Step 4: Check for next intersection point and find its Y-value, and compare with Y-max. If it's equal to Y-max (+/- a threshold), evaluate the next respiration cycle (starting from step 2).

A second waveform image 2020 shows a double trigger, where no expiratory flow occurs between inspiratory flows. A variation or extension of the twelfth comparison model may be employed to identify the two closest points of intersection with the x axis and determine the distance between the two closest points. For example, the second output shows the two points of intersection that are closest to one another. The distance between these two intersection points may be calculated, and if the distance is less than a threshold, double triggering may be identified.

FIG. 21 shows a fourth set of waveform images 2100 that may be evaluated to determine a change in patient ventilation status. The fourth set of waveform images 2100 may be generated with the medical system 10 during execution of one or more of the methods described above with respect to FIGS. 4-7 and analyzed via the one or more comparison models (e.g., the comparison models of the model repository of FIG. 3). The fourth set of waveform images 2100 includes a pressure waveform image 2110, a flow waveform image 2120, and a volume waveform image 2130, each obtained of the same patient simultaneously.

The fourth set of waveform images 2100 includes features indicative of the patient attempting to breathe spontaneously. For example, the patient initiates a breath mid-inspiratory cycle in the first illustrated respiration cycle, which is evidenced by a negative pressure deflection in the pressure waveform image 2110, a decrease in flow (due to the patient effort) in the flow waveform image 2120, and a change in delivered volume due to patient inspiratory effort in the volume waveform image 2130. Additionally, the patient initiates another breath that causes the inspiratory valve to remain closed, and thus no flow is provided for the subsequent respiratory cycle. As a result, a negative pressure deflection is observed in the pressure waveform image 2110 and missed triggers are observed in the flow waveform image 2120 and the volume waveform image 2130. Further, the patient attempts to exhale during a subsequent inspiratory cycle, which creates positive pressure (shown on the pressure waveform image 2110) and causes the expiratory valve to remain closed. The flow waveform image 2120 illustrates expiratory flow with patient exhalation effort and the volume waveform image 2130 illustrates tidal volume decrease due to the patient exhalation effort.

Spontaneous breathing may be detected with the comparison models disclosed herein based on the detection of a small negative pressure before the next breathing cycle. The process of detecting spontaneous breathing may include obtaining the baseline image, rescaling the current image to the same size as the baseline image if needed, comparing point to point deviation, and comparing overall deviation.

Thus, the example comparison models described above may be employed to identify and quantify features of current patient ventilation parameter images and reference ventilation parameter images (e.g., patient baseline images or selected annotated/ground truth images), and deviations between features in the current patient ventilation parameter images and corresponding features in the reference ventilation parameter images may trigger one or more notifications to be output in order to alert one or more clinicians monitoring the patient that the patient status has changed. The comparison models may employ various thresholding, segmentation, and clustering techniques, such as color segmentation, edge segmentation, adaptive thresholding, standard deviation determination, bit manipulation, edge detection, contour detection, and density-based clustering to identify and quantify the features in the images, as explained in the examples above. While at least some of the features of the ventilation parameter images may be characterized/quantified with other techniques, the comparison models disclosed herein may provide for robust feature characterization with minimal error, compared to other techniques.

Still further features may be identified and quantified with the comparison models. For example, on volume waveform images, the area under the curve may be determined for each of the inspiratory and expiratory phases. The inspiratory volume should be equal to the expiratory volume, and differences between the volumes can indicate air leaks in the system or intrinsic positive end-expiratory pressure (e.g., auto-PEEP or air trapping). Additionally, the flow waveform images can provide useful information about a patient's exhalation. The shape of the expiratory limb of the flow waveform is affected by the resistance to air flow and the compliance of the lung. In instances of higher airway resistance from some obstructive process, the flow waveform will show a decreased peak expiratory flow and a prolonged time for the expiratory curve to return to a baseline of zero flow.

The pressure waveform images and pressure spirometry images (e.g., pressure volume) may provide information about airway compliance. Regardless of mode the Pplat is measured in (e.g., pressure targeted mode or volume control or flow targeted control), a large difference between the PIP and the Pplat as seen in the pressure waveforms indicates high resistance in the airway as can exemplified in severe bronchospasm. Further, in normally compliant lungs, pressure rises at a constant rate with a constant rise in volume. This equates to a stress index of one and a straight slope for the segment of the pressure/volume spirometry loop where volume increases during expiration. A decrease in the slope is consistent with a stress index less than one, giving it a downward curved shape. This suggests improvement in compliance and recruitment of the lung with increasing volume. An increase in the slope resulting in an upward curved shape, or a stress index greater than one, is seen when the lung compliance is worse with a rise in volume. This may indicate over-distension of the lung, which may be detected with the seventh comparison model and is shown in FIG. 14.

Several important pieces of information about air flow in and out of the lungs can be obtained from evaluation of the flow-volume spirometry images, particularly the expiratory limb. For example, the flow-volume loop provides for measurement of a peak expiratory flow rate. A lower peak expiratory flow rate indicates potential obstruction, such as can be seen with bronchoconstriction.

It is to be appreciated that some of the features discussed above may be specific to spirometry images while other features may be specific to waveform images. Still other features may be common to both waveform and spirometry images. As such, some but not all of the comparison models described above may be invoked when a specific ventilator parameter image is processed to characterize/quantify the features in that ventilation parameter image (e.g., the comparison models applied to a given ventilation parameter image may be selected based on the type of image, such as waveform or spirometry image). Further, each possible feature (e.g., as described above) may be characterized for a given ventilator parameter image, or only selected features may be characterized. Thus, the comparison between a current patient ventilation parameter image and a corresponding reference ventilation parameter image may include characterizing at least one feature of each image using at least one comparison model. Further still, it is to be appreciated that the separate comparison models described above may be combined as appropriate in order to form larger models configured to characterize multiple features in a given ventilation parameter image. However, by providing separate comparison models as described above with respect to FIGS. 9-21, each configured to perform an image processing task(s) to facilitate characterization of a feature(s), various benefits may be achieved, such as ability to perform parallel processing on a given ventilation parameter image, update or add new comparison models as appropriate without affecting performance of the remaining comparison models, and invoke only those comparison models deemed appropriate for the given ventilation parameter image.

A technical effect of comparing, with at least one comparison model, each patient ventilation parameter image of a plurality of patient ventilation parameter images with a respective reference ventilation parameter image is that deviations from the reference ventilation parameter images may be automatically detected so that one or more clinicians can be alerted of the deviation in order to adjust ventilation system settings, if indicated. Patients may be ventilated for hours, days, or even weeks, and thus another technical effect of automatically detecting deviations from the reference ventilation parameter images is that deviations that might otherwise go unnoticed if only a clinician were monitoring the patient ventilation parameter images may be detected as soon as the deviation occurs. The reference ventilation parameter images may be ground truth images obtained of other patients or the reference ventilation parameter images may be prior images of the patient. In each case, the reference ventilation parameter images may represent target or ideal ventilation parameters for the patient, a deviation from which may signal an issue with the patient or ventilation system.

The disclosure also provides support for a method for a ventilation system, comprising: obtaining one or more patient ventilation parameter images of a patient with the ventilation system while the patient is undergoing mechanical ventilation with the ventilation system, obtaining one or more reference ventilation parameter images, processing, with at least one comparison model, each patient ventilation parameter image and each reference ventilation parameter image to characterize at least one feature in each patient ventilation parameter image and each reference ventilation parameter image, the processing including converting each patient ventilation parameter image and each reference ventilation parameter image to a binary mask, identifying, based on the at least one feature in each patient ventilation parameter image and each reference ventilation parameter image, a deviation between a patient ventilation parameter image and a corresponding reference ventilation parameter image, and in response to the identifying, outputting a notification that indicates the deviation. In a first example of the method, the one or more reference ventilation parameter images are of one or more prior patients and/or of an artificial lung, and wherein the one or more reference ventilation parameter images are selected from a data store of ground truth ventilation parameter images based on patient information of the patient and/or settings of the ventilation system while the patient is undergoing mechanical ventilation. In a second example of the method, optionally including the first example, the one or more patient ventilation parameter images comprise one or more current patient ventilation parameter images of the patient, and wherein each reference ventilation parameter image is a respective baseline ventilation parameter image of the patient obtained prior to the obtaining of the one or more current patient ventilation parameter images. In a third example of the method, optionally including one or both of the first and second examples, the method further comprises: storing a prior patient ventilation parameter image as a respective baseline ventilation parameter image in response to the prior patient ventilation parameter image not deviating from a selected ventilation parameter image, the selected ventilation parameter image obtained from a data store of ground truth ventilation parameter images based on patient information of the patient and/or settings of the ventilation system. In a fourth example of the method, optionally including one or more or each of the first through third examples, the one or more patient ventilation parameter images comprise one or more of a pressure waveform image, a flow waveform image, and a volume waveform image. In a fifth example of the method, optionally including one or more or each of the first through fourth examples, the one or more patient ventilation parameter images comprise one or more of a pressure/volume spirometry image and a flow/volume spirometry image. In a sixth example of the method, optionally including one or more or each of the first through fifth examples, identifying the deviation comprises identifying that the patient is breathing spontaneously, and in response, executing a weaning protocol to determine a readiness of the patient to be weaned from the mechanical ventilation. In a seventh example of the method, optionally including one or more or each of the first through sixth examples, executing the weaning protocol comprises outputting an audio command to the patient, recording a video of the patient during the outputting of the audio command, and analyzing the video, via a selected comparison model of the at least one comparison model, to determine if the patient has obeyed the audio command. In an eighth example of the method, optionally including one or more or each of the first through seventh examples, the method further comprises: obtaining one or more further patient ventilation parameter images of the patient, processing, with the at least one comparison model, each further patient ventilation parameter image and a respective reference ventilation parameter image that corresponds to each further patient ventilation parameter image to characterize the at least one feature in each further patient ventilation parameter image and each respective reference ventilation parameter image, determining, based on the at least one feature of each further patient ventilation parameter image and each respective reference ventilation parameter image, that a change in settings of the ventilation system has been made, and in response to the determining, setting the one or more further patient ventilation parameter images as the one or more reference ventilation parameter images. In a ninth example of the method, optionally including one or more or each of the first through eighth examples, the at least one comparison model is configured to perform color segmentation, edge segmentation, adaptive thresholding, standard deviation determination, bit manipulation, edge detection, contour detection, density-based clustering, and combinations thereof. In some examples, a ventilation system is configured to perform the method and optionally one or more or each of the first through ninth examples.

The disclosure also provides support for a ventilation system, comprising: memory storing instructions, and one or more processors configured to execute the instructions in order to: obtain one or more patient ventilation parameter images of a patient with the ventilation system while the patient is undergoing mechanical ventilation with the ventilation system, obtain one or more reference ventilation parameter images, compare, with at least one comparison model, each patient ventilation parameter image with a respective reference ventilation parameter image, the comparing including comparing at least one feature in each patient ventilation parameter image to a corresponding feature in the respective ventilation parameter image, each feature characterized with the at least one comparison model after conversion of each patient ventilation parameter image and each reference ventilation parameter image to a respective binary mask, and in response to determining, based on the comparing, that at least one of the one or more patient ventilation parameter images deviates from a corresponding reference ventilation parameter image, output a notification that indicates the deviation and a probable cause of the deviation. In a first example of the system, the one or more patient ventilation parameter images comprise one or more of a pressure waveform image, a flow waveform image, a volume waveform image, a pressure/volume spirometry image, and a flow/volume spirometry image. In a second example of the system, optionally including the first example, the one or more reference ventilation parameter images are of one or more prior patients and/or of an artificial lung, and wherein the one or more reference ventilation parameter images are selected from a data store of ground truth ventilation parameter images based on patient information of the patient and/or settings of the ventilation system while the patient is undergoing mechanical ventilation. In a third example of the system, optionally including one or both of the first and second examples, the one or more patient ventilation parameter images comprise one or more current patient ventilation parameter images of the patient, and wherein each reference ventilation parameter image is a respective baseline ventilation parameter image of the patient obtained prior to the obtaining of the one or more current patient ventilation parameter images. In a fourth example of the system, optionally including one or more or each of the first through third examples, the instructions are further executable to store a prior patient ventilation parameter image as a respective baseline ventilation parameter image in response to the prior patient ventilation parameter image not deviating from a selected ventilation parameter image, the selected ventilation parameter image obtained from a data store of ground truth ventilation parameter images based on patient information of the patient and/or settings of the ventilation system.

The disclosure also provides support for a method, comprising: obtaining a patient ventilation parameter image of a patient with a ventilation system while the patient is undergoing mechanical ventilation with the ventilation system, comparing, based on output from at least one comparison model, the patient ventilation parameter image with a baseline ventilation parameter image of the patient obtained prior to obtaining the patient ventilation parameter image, wherein at least one of the at least one comparison model is configured to convert the patient ventilation parameter image and the baseline ventilation parameter image to a respective binary mask using color segmentation, identifying, based on the comparing, a deviation between the patient ventilation parameter image and the baseline ventilation parameter image, in response to the determining, outputting a notification that indicates the deviation, obtaining a subsequent patient ventilation parameter image of the patient with the ventilation system while the patient continues to undergo mechanical ventilation with the ventilation system, and determining, based on the subsequent patient ventilation parameter image, that one or more settings of the ventilation system have been changed, and in response, replacing the baseline ventilation parameter image with the subsequent patient ventilation parameter image. In a first example of the method, the method further comprises: storing a prior patient ventilation parameter image as the baseline ventilation parameter image in response to determining, via the at least one comparison model, that the prior patient ventilation parameter image does not deviate from a selected ventilation parameter image, the selected ventilation parameter image obtained from a data store of ground truth ventilation parameter images based on patient information of the patient and/or settings of the ventilation system. In a second example of the method, optionally including the first example, the patient ventilation parameter image comprises a pressure waveform image, a flow waveform image, a volume waveform image, a pressure/volume spirometry image, or a flow/volume spirometry image. In a third example of the method, optionally including one or both of the first and second examples, comparing, with the at least one comparison model, the patient ventilation parameter image with the baseline ventilation parameter image comprises: performing, with the at least one comparison model, one or more of color segmentation, edge segmentation, adaptive thresholding, standard deviation determination, bit manipulation, edge detection, contour detection, and density-based clustering to quantify one or more features in the patient ventilation parameter image, comparing each feature of the one or more features in the patient ventilation parameter image to a respective corresponding feature in the baseline ventilation parameter image, and identifying, based on the comparing, the deviation between the patient ventilation parameter image and the baseline ventilation parameter image based on at least one feature of the one or more features differing from its corresponding feature by at least a threshold amount. In a fourth example of the method, optionally including one or more or each of the first through third examples, the patient ventilation parameter image depicts a plot of a first patient ventilation parameter as a function of time or as a function of a second patient ventilation parameter, and wherein the one or more features comprises one or more of an area enclosed by a loop in the plot, a number of points of intersection with an x axis and/or a y axis of the plot, a hysteresis elongation of the plot, a curvature of a segment of the loop in the plot, and an angle of another segment of the loop in the plot. In some examples, a ventilation system is configured to perform the method and optionally one or more or each of the first through fourth examples.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method for a ventilation system, comprising:
obtaining (504, 602) one or more patient ventilation parameter images of a patient with the ventilation system while the patient is undergoing mechanical ventilation with the ventilation system;
obtaining one or more reference ventilation parameter images;
processing, with at least one comparison model, each patient ventilation parameter image and each reference ventilation parameter image to characterize at least one feature in each patient ventilation parameter image and each reference ventilation parameter image, the processing including converting each patient ventilation parameter image and each reference ventilation parameter image to a binary mask;
identifying (506, 508, 604, 606), based on the at least one feature in each patient ventilation parameter image and each reference ventilation parameter image, a deviation between a patient ventilation parameter image and a corresponding reference ventilation parameter image; and
in response to the identifying, outputting (510, 608) a notification that indicates the deviation.

2. The method of claim 1, wherein the one or more reference ventilation parameter images are of one or more prior patients and/or of an artificial lung, and wherein the one or more reference ventilation parameter images are selected from a data store of ground truth ventilation parameter images based on patient information of the patient and/or settings of the ventilation system while the patient is undergoing mechanical ventilation.

3. The method of claim 1, wherein the one or more patient ventilation parameter images comprise one or more current patient ventilation parameter images of the patient, and wherein each reference ventilation parameter image is a respective baseline ventilation parameter image of the patient obtained prior to the obtaining of the one or more current patient ventilation parameter images.

4. The method of claim 3, further comprising storing (512, 618) a prior patient ventilation parameter image as a respective baseline ventilation parameter image in response to the prior patient ventilation parameter image not deviating from a selected ventilation parameter image, the selected ventilation parameter image obtained from a data store of ground truth ventilation parameter images based on patient information of the patient and/or settings of the ventilation system.

5. The method of claim 1, wherein the one or more patient ventilation parameter images comprise one or more of a pressure waveform image, a flow waveform image, and a volume waveform image.

6. The method of claim 1, wherein the one or more patient ventilation parameter images comprise one or more of a pressure/volume spirometry image and a flow/volume spirometry image.

7. The method of claim 1, wherein identifying the deviation comprises identifying (626) that the patient is breathing spontaneously, and in response, executing a weaning protocol (700) to determine a readiness of the patient to be weaned from the mechanical ventilation.

8. The method of claim 7, wherein executing the weaning protocol comprises outputting (702) an audio command to the patient, recording (704) a video of the patient during the outputting of the audio command, and analyzing the video, via a selected comparison model of the at least one comparison model, to determine if the patient has obeyed the audio command.

9. The method of claim 1, further comprising:
obtaining (610) one or more further patient ventilation parameter images of the patient;
processing, with the at least one comparison model, each further patient ventilation parameter image and a respective reference ventilation parameter image that corresponds to each further patient ventilation parameter image to characterize the at least one feature in each further patient ventilation parameter image and each respective reference ventilation parameter image;
determining (614), based on the at least one feature of each further patient ventilation parameter image and each respective reference ventilation parameter image, that a change in settings of the ventilation system has been made; and
in response to the determining, setting (618) the one or more further patient ventilation parameter images as the one or more reference ventilation parameter images.

10. The method of claim 1, wherein the at least one comparison model is configured to perform color segmentation, edge segmentation, adaptive thresholding, standard deviation determination, bit manipulation, edge detection, contour detection, density-based clustering, and combinations thereof.

11. A ventilation system (100), comprising:
memory (102) storing instructions; and
one or more processors (58) configured to execute the instructions in order to:
obtain (504, 602) one or more patient ventilation parameter images of a patient with the ventilation system while the patient is undergoing mechanical ventilation with the ventilation system;
obtain one or more reference ventilation parameter images;
compare (506, 604), with at least one comparison model, each patient ventilation parameter image with a respective reference ventilation parameter image, the comparing including comparing at least one feature in each patient ventilation parameter image to a corresponding feature in the respective ventilation parameter image, each feature characterized with the at least one comparison model after conversion of each patient ventilation parameter image and each reference ventilation parameter image to a respective binary mask; and
in response to determining, based on the comparing, that at least one of the one or more patient ventilation parameter images deviates from a corresponding reference ventilation parameter image, output (510, 608) a notification that indicates the deviation and a probable cause of the deviation.

12. The ventilation system of claim 11, wherein the one or more patient ventilation parameter images comprise one or more of a pressure waveform image, a flow waveform image, a volume waveform image, a pressure/volume spirometry image, and a flow/volume spirometry image.

13. The ventilation system of claim 11, wherein the one or more reference ventilation parameter images are of one or more prior patients and/or of an artificial lung, and wherein the one or more reference ventilation parameter images are selected from a data store (116) of ground truth ventilation parameter images based on patient information of the patient and/or settings of the ventilation system while the patient is undergoing mechanical ventilation.

14. The ventilation system of claim 11, wherein the one or more patient ventilation parameter images comprise one or more current patient ventilation parameter images of the patient, and wherein each reference ventilation parameter image is a respective baseline ventilation parameter image of the patient obtained prior to the obtaining of the one or more current patient ventilation parameter images.

15. The ventilation system of claim 14, wherein the instructions are further executable to store (512) a prior patient ventilation parameter image as a respective baseline ventilation parameter image in response to the prior patient ventilation parameter image not deviating from a selected ventilation parameter image, the selected ventilation parameter image obtained from a data store (116) of ground truth ventilation parameter images based on patient information of the patient and/or settings of the ventilation system.
